# EUROPEAN PATENT APPLICATION

(11) **EP 3 407 071 A1**
(43) Date of publication of application: **28.11.2018**
(21) Application number: 17741038.8
(22) Date of filing: 18.01.2017
(51) Int. Cl.: G01N 33/68

(54) **METHOD AND KIT FOR DIAGNOSIS OF ACTIVE TUBERCULOSIS**

(30) Priority: 20.01.2016 CN 201610034476
(71) Applicant: Xiamen University, Xiamen, Fujian 361005 (CN); Beijing Wantai Biological Pharmacy Enterprise Co., Ltd., Changping District Beijing 102206 (CN)
(72) Inventor: GE, Shengxiang, Xiamen Fujian 361005 (CN); LIU, Yongliang, Xiamen Fujian 361005 (CN); CHEN, Mengyuan, Xiamen Fujian 361005 (CN); ZHANG, Jun, Xiamen Fujian 361005 (CN); XIA, Ningshao, Xiamen Fujian 361005 (CN)
(74) Representative: Lavoix
(86) International application number: PCT/CN2017/071462
(87) International publication number: WO 2017/125007

(57) **Abstract**

Disclosed are a method for diagnosing whether a subject is suffering from active tuberculosis, a method for determining the therapeutic effect of a therapy on active tuberculosis, a method for screening a candidate drug capable of treating active tuberculosis, and a kit comprising a specific stimulating agent and a reagent for detecting the level of IL-6, the method and kit belong to the fields of molecular biology, immunology and disease diagnosis.

SEQUENCE LISTING

<110>  XIAMEN UNIVERSITY
       BEIJING WANTAI BIOLOGICAL PHARMACY ENTERPRISE CO., LTD.

<120>  Method and kit for diagnosis of active tuberculosis

<130>  BET 18M2880

<140>  EP17741038.8
<141>  2017-01-18

<150>  CN201610034476.6
<151>  2016-01-20

<160>  29

<170>  PatentIn version 3.5

<210>  1
<211>  279
<212>  PRT
<213>  Mycobacterium tuberculosis

<400>  1

Met Thr Thr Thr Arg Thr Glu Arg Asn Phe Ala Gly Ile Gly Asp Val
1               5                   10                  15

Arg Ile Val Tyr Asp Val Trp Thr Pro Asp Thr Ala Pro Gln Ala Val
            20                  25                  30

Val Val Leu Ala His Gly Leu Gly Glu His Ala Arg Arg Tyr Asp His
        35                  40                  45

Val Ala Gln Arg Leu Gly Ala Ala Gly Leu Val Thr Tyr Ala Leu Asp
    50                  55                  60

His Arg Gly His Gly Arg Ser Gly Gly Lys Arg Val Leu Val Arg Asp
65                  70                  75                  80

Ile Ser Glu Tyr Thr Ala Asp Phe Asp Thr Leu Val Gly Ile Ala Thr
                85                  90                  95

Arg Glu Tyr Pro Gly Cys Lys Arg Ile Val Leu Gly His Ser Met Gly
            100                 105                 110

Gly Gly Ile Val Phe Ala Tyr Gly Val Glu Arg Pro Asp Asn Tyr Asp
        115                 120                 125

Leu Met Val Leu Ser Ala Pro Ala Val Ala Ala Gln Asp Leu Val Ser
    130                 135                 140

Pro Val Val Ala Val Ala Ala Lys Leu Leu Gly Val Val Val Pro Gly

145                 150                 155                 160

Leu Pro Val Gln Glu Leu Asp Phe Thr Ala Ile Ser Arg Asp Pro Glu
                165                 170                 175

Val Val Gln Ala Tyr Asn Thr Asp Pro Leu Val His His Gly Arg Val
            180                 185                 190

Pro Ala Gly Ile Gly Arg Ala Leu Leu Gln Val Gly Glu Thr Met Pro
        195                 200                 205

Arg Arg Ala Pro Ala Leu Thr Ala Pro Leu Leu Val Leu His Gly Thr
    210                 215                 220

Asp Asp Arg Leu Ile Pro Ile Glu Gly Ser Arg Arg Leu Val Glu Cys
225                 230                 235                 240

Val Gly Ser Ala Asp Val Gln Leu Lys Glu Tyr Pro Gly Leu Tyr His
                245                 250                 255

Glu Val Phe Asn Glu Pro Glu Arg Asn Gln Val Leu Asp Asp Val Val
            260                 265                 270

Ala Trp Leu Thr Glu Arg Leu
        275

<210>  2
<211>  852
<212>  DNA
<213>  Mycobacterium tuberculosis

<400>  2
ggatccatga ctaccacccg gactgaacgg aatttcgcgg gcatcggcga tgtgcgcatc      60

gtctacgacg tctggacgcc ggacaccgcg ccgcaagcgg tggtcgtgct ggcccatggt     120

ctgggcgagc atgcccgccg ctacgaccat gtcgcgcagc ggctcggcgc ggccggcctg     180

gtcacctatg cgcttgacca ccgcgggcat ggccgctcgg gtggcaaacg ggtgctagtg     240

agagacatct ccgagtacac cgctgacttc gacaccctcg ttgggatcgc cacccgggaa     300

tatcccgggt gcaagcgcat cgtgctcggg cacagcatgg gcggcggcat tgtgttcgct     360

tacggtgtcg aacgtccaga caactacgac ctgatggtgc tttcggcgcc ggcggtggcg     420

gcacaggacc tggtgagccc ggtagtggcg gttgccgcca agcttctggg cgtcgtggtg     480

cccggcctgc cggtgcagga actggatttt actgccatct ctcgcgaccc tgaggtggtc     540

caggcttaca acaccgaccc actcgtgcac cacggacggg ttccggccgg gattggccgc     600

gcgctgctgc aggtgggcga gaccatgccg cggcgagcac cggcattgac cgcgccgctg     660

ctagtgctgc acggcaccga tgaccggctg atccccatcg agggcagccg tcgcctggtc 720

gaatgtgtgg gatcggccga cgtgcagctg aaggagtatc ccgggctgta ccacgaggtg 780

ttcaacgagc cggagcgcaa ccaggtgctc gacgatgtgg tcgcctggct caccgagcgg 840

ttgtaagaat tc 852

<210> 3
<211> 280
<212> PRT
<213> Mycobacterium tuberculosis

<400> 3

Asp Ala Gly Val Ser Trp Lys Val Tyr Arg Asn Lys Thr Leu Gly Pro
1               5                   10                  15

Ile Ser Ser Val Leu Thr Tyr Gly Ser Leu Val Thr Ser Phe Lys Gln
            20                  25                  30

Ser Ala Asp Pro Arg Ser Asp Leu Val Arg Phe Gly Val Ala Pro Ser
        35                  40                  45

Tyr Pro Ala Ser Phe Ala Ala Asp Val Leu Ala Asn Arg Leu Pro Arg
    50                  55                  60

Val Ser Trp Val Ile Pro Asn Val Leu Glu Ser Glu His Pro Ala Val
65                  70                  75                  80

Pro Ala Ala Ala Gly Ala Phe Ala Ile Val Asn Ile Leu Arg Ile Leu
                85                  90                  95

Leu Ala Asn Pro Ala Val Trp Glu Lys Thr Ala Leu Ile Val Ser Tyr
            100                 105                 110

Asp Glu Asn Gly Gly Phe Phe Asp His Val Val Pro Ala Thr Ala Pro
        115                 120                 125

Ala Gly Thr Pro Gly Glu Tyr Val Thr Val Pro Asp Ile Asp Gln Val
    130                 135                 140

Pro Gly Ser Gly Gly Ile Arg Gly Pro Ile Gly Leu Gly Phe Arg Val
145                 150                 155                 160

Pro Cys Phe Val Ile Ser Pro Tyr Ser Arg Gly Pro Gln Met Val His
                165                 170                 175

Asp Thr Phe Asp His Thr Ser Gln Leu Arg Leu Leu Glu Thr Arg Phe
            180                 185                 190

Gly Val Pro Val Pro Asn Leu Thr Ala Trp Arg Arg Ser Val Thr Gly
        195                 200                 205

Asp Met Thr Ser Thr Phe Asn Phe Ala Val Pro Pro Asn Ser Ser Trp
    210                 215                 220

Pro Asn Leu Asp Tyr Pro Gly Leu His Ala Leu Ser Thr Val Pro Gln
225                 230                 235                 240

Cys Val Pro Asn Ala Ala Leu Gly Thr Ile Asn Arg Gly Ile Pro Tyr
                245                 250                 255

Arg Val Pro Asp Pro Gln Ile Met Pro Thr Gln Glu Thr Thr Pro Thr
            260                 265                 270

Arg Gly Ile Pro Ser Gly Pro Cys
        275                 280

<210>  4
<211>  843
<212>  DNA
<213>  Mycobacterium tuberculosis

<400>  4
gatgccggcg tcagctggaa ggtgtatcgc aacaagacac tcgggcccat ctcctcggtt      60

cttacttacg gctcgcttgt gacgtctttc aaacagtcag ccgatcccag gtcagatctt     120

gtccgctttg gcgtggcacc aagctatccc gcgagcttcg cggccgacgt cttagccaat     180

agactgccgc gggtctcctg ggtgattccc aatgttctcg aatccgaaca tcctgcggtt     240

ccagccgcgg ccggggcttt cgcaatcgtc aacatcttaa gaatattgct tgccaatcct     300

gcggtgtggg aaaagacggc gctgatcgtc agctacgacg aaaacggcgg ctttttcgac     360

cacgttgttc ctgctaccgc gccggccggg actcccggcg aatatgtcac ggtgcctgac     420

atcgatcagg tgccgggctc cggcggaata cgcgggccga tcggtttggg ctttcgcgtt     480

ccctgcttcg tcatttcgcc gtacagccgt ggcccgcaga tggttcacga cacgtttgac     540

cacacctcac agctgagatt gctcgaaact cggttcgggg tgccagttcc caacctcacg     600

gcttggcggc gcagtgtgac cggcgacatg acgtcaacgt tcaacttcgc tgtcccgccc     660

aactcatcat ggcccaacct ggattatccc gggctgcacg cgctatcaac ggtgccgcag     720

tgcgtgccca acgcggcgct gggcacgata aaccgtggaa tcccgtatcg ggttcctgat     780

ccacagatca tgcccacgca ggaaaccacg cctacccgtg gtattccgag cggtccgtgc     840

taa                                                                   843

<210>  5

<211>  20
<212>  PRT
<213>  artificial sequence

<220>
<223>  RV0183-p1

<400>  5

Met Thr Thr Thr Arg Thr Glu Arg Asn Phe Ala Gly Ile Gly Asp Val
1               5                   10                  15


Arg Ile Val Tyr
            20


<210>  6
<211>  20
<212>  PRT
<213>  artificial sequence

<220>
<223>  RV0183-p2

<400>  6

Gly Asp Val Arg Ile Val Tyr Asp Val Trp Thr Pro Asp Thr Ala Pro
1               5                   10                  15


Gln Ala Val Val
            20


<210>  7
<211>  20
<212>  PRT
<213>  artificial sequence

<220>
<223>  RV0183-p3

<400>  7

Thr Ala Pro Gln Ala Val Val Val Leu Ala His Gly Leu Gly Glu His
1               5                   10                  15


Ala Arg Arg Tyr
            20


<210>  8
<211>  20
<212>  PRT
<213>  artificial sequence

<220>
<223>  RV0183-p4

<400>  8

Gly Glu His Ala Arg Arg Tyr Asp His Val Ala Gln Arg Leu Gly Ala
1               5                   10                  15

Ala Gly Leu Val
            20

<210>  9
<211>  20
<212>  PRT
<213>  artificial sequence

<220>
<223>  RV0183-p5

<400>  9

Leu Gly Ala Ala Gly Leu Val Thr Tyr Ala Leu Asp His Arg Gly His
1               5                   10                  15

Gly Arg Ser Gly
            20

<210>  10
<211>  20
<212>  PRT
<213>  artificial sequence

<220>
<223>  RV0183-p6

<400>  10

Arg Gly His Gly Arg Ser Gly Gly Lys Arg Val Leu Val Arg Asp Ile
1               5                   10                  15

Ser Glu Tyr Thr
            20

<210>  11
<211>  20
<212>  PRT
<213>  artificial sequence

<220>
<223>  RV0183-p7

<400>  11

Arg Asp Ile Ser Glu Tyr Thr Ala Asp Phe Asp Thr Leu Val Gly Ile
1               5                   10                  15

Ala Thr Arg Glu
            20

<210>  12

<211>  20
<212>  PRT
<213>  artificial sequence

<220>
<223>  RV0183-p8

<400>  12

Val Gly Ile Ala Thr Arg Glu Tyr Pro Gly Cys Lys Arg Ile Val Leu
1               5                   10                  15

Gly His Ser Met
            20

<210>  13
<211>  20
<212>  PRT
<213>  artificial sequence

<220>
<223>  RV0183-p9

<400>  13

Ile Val Leu Gly His Ser Met Gly Gly Gly Ile Val Phe Ala Tyr Gly
1               5                   10                  15

Val Glu Arg Pro
            20

<210>  14
<211>  20
<212>  PRT
<213>  artificial sequence

<220>
<223>  RV0183-p10

<400>  14

Ala Tyr Gly Val Glu Arg Pro Asp Asn Tyr Asp Leu Met Val Leu Ser
1               5                   10                  15

Ala Pro Ala Val
            20

<210>  15
<211>  20
<212>  PRT
<213>  artificial sequence

<220>
<223>  RV0183-p11

<400>  15

Val Leu Ser Ala Pro Ala Val Ala Ala Gln Asp Leu Val Ser Pro Val
1               5                   10                  15

Val Ala Val Ala
            20

<210>  16
<211>  20
<212>  PRT
<213>  artificial sequence

<220>
<223>  RV0183-p12

<400>  16

Ser Pro Val Val Ala Val Ala Ala Lys Leu Leu Gly Val Val Val Pro
1               5                   10                  15

Gly Leu Pro Val
            20

<210>  17
<211>  20
<212>  PRT
<213>  artificial sequence

<220>
<223>  RV0183-p13

<400>  17

Val Val Pro Gly Leu Pro Val Gln Glu Leu Asp Phe Thr Ala Ile Ser
1               5                   10                  15

Arg Asp Pro Glu
            20

<210>  18
<211>  20
<212>  PRT
<213>  artificial sequence

<220>
<223>  RV0183-p14

<400>  18

Ala Ile Ser Arg Asp Pro Glu Val Val Gln Ala Tyr Asn Thr Asp Pro
1               5                   10                  15

Leu Val His His
            20

<210>  19

<211>  20
<212>  PRT
<213>  artificial sequence

<220>
<223>  RV0183-p15

<400>  19

Thr Asp Pro Leu Val His His Gly Arg Val Pro Ala Gly Ile Gly Arg
1               5                   10                  15

Ala Leu Leu Gln
            20

<210>  20
<211>  20
<212>  PRT
<213>  artificial sequence

<220>
<223>  RV0183-p16

<400>  20

Ile Gly Arg Ala Leu Leu Gln Val Gly Glu Thr Met Pro Arg Arg Ala
1               5                   10                  15

Pro Ala Leu Thr
            20

<210>  21
<211>  20
<212>  PRT
<213>  artificial sequence

<220>
<223>  RV0183-p17

<400>  21

Arg Arg Ala Pro Ala Leu Thr Ala Pro Leu Leu Val Leu His Gly Thr
1               5                   10                  15

Asp Asp Arg Leu
            20

<210>  22
<211>  20
<212>  PRT
<213>  artificial sequence

<220>
<223>  RV0183-p18

<400>  22

His Gly Thr Asp Asp Arg Leu Ile Pro Ile Glu Gly Ser Arg Arg Leu
1               5                   10                  15

Val Glu Cys Val
            20

<210>  23
<211>  20
<212>  PRT
<213>  artificial sequence

<220>
<223>  RV0183-p19

<400>  23

Arg Arg Leu Val Glu Cys Val Gly Ser Ala Asp Val Gln Leu Lys Glu
1               5                   10                  15

Tyr Pro Gly Leu
            20

<210>  24
<211>  20
<212>  PRT
<213>  artificial sequence

<220>
<223>  RV0183-p20

<400>  24

Leu Lys Glu Tyr Pro Gly Leu Tyr His Glu Val Phe Asn Glu Pro Glu
1               5                   10                  15

Arg Asn Gln Val
            20

<210>  25
<211>  19
<212>  PRT
<213>  artificial sequence

<220>
<223>  RV0183-p21

<400>  25

Glu Pro Glu Arg Asn Gln Val Leu Asp Asp Val Val Ala Trp Leu Thr
1               5                   10                  15

Glu Arg Leu

<210>  26

<211>  23
<212>  DNA
<213>  artificial sequence

<220>
<223>  primer

<400>  26
ttcaaccatc gccgcctcta cca                                              23


<210>  27
<211>  20
<212>  DNA
<213>  artificial sequence

<220>
<223>  primer

<400>  27
ccatcgccgc ctctaccagt                                                  20


<210>  28
<211>  23
<212>  DNA
<213>  artificial sequence

<220>
<223>  primer

<400>  28
ggatccatgg atgccggcgt cag                                              23


<210>  29
<211>  23
<212>  DNA
<213>  artificial sequence

<220>
<223>  primer

<400>  29
aagcttttag cacggaccgc tcg                                              23

## Description

### Technical Field

The invention belongs to the fields of molecular biology, immunology and disease diagnosis. In particular, the invention relates to a method for diagnosing whether a subject is suffering from active tuberculosis, a method for determining the therapeutic effect of a therapy on active tuberculosis, and a method for screening a candidate drug capable of treating active tuberculosis. The invention further relates to a kit comprising a specific stimulating agent and a reagent for detecting the level of IL-6.

### Background Art

Tuberculosis (TB), a chronic infectious disease caused by *Mycobacterium tuberculosis* (*M*. *tuberculosis*) infection, is a very serious health problem around the world. There are about 9 million new TB patients every year over the world, and up to 1.7 million people died of tuberculosis. In addition, it is estimated that about one-third of the world's population is latently infected with *M. tuberculosis,* which is the source of potential active tuberculosis (aTB) patients.

To control tuberculosis, the foremost thing is to discover and cure patients with infectious tuberculosis. After X-ray has been applied in clinical tests, it provides a simple, convenient, quick, and highly sensitive method for discovering TB patients. However, X-ray detection has disadvantages such as high demands on equipment, low specificity, and a high rate of over-diagnosis, missed diagnosis, and misdiagnosis. In addition, X-ray detection can only detect the suspicious shadows in lungs, but cannot achieve the purpose of detecting infectious patients. Therefore, it is explicitly pointed out in the 9^{th} report of the WHO Expert Committee on Tuberculosis (1974) that active finding of patients by chest X-ray examination in a population is not feasible, and has to be abolished not only in developed countries but also in developing countries.

The sputum smear test can detect infectious TB patients, and has advantages such as high specificity, simple equipment, low cost, and low demands on techniques. However, there are also problems concerning difficulty in sputum collection; for example, many patients suffer from dry cough without sputum, or a trace amount of bacteria excreted in sputum is not easy to be detected. The sensitivity of widely used sputum smear tests is between 34% and 80%. Although sputum culture test has a higher sensitivity than sputum smear test, it takes a long time and has high demands on diagnostic laboratories. Many countries with high burdens of tuberculosis cannot meet the laboratory conditions required for the method. Immunoassay technologies may have a great application value in the diagnosis of tuberculosis, especially after the realization of rapid diagnosis and bedside diagnosis.

IFN-γ release assays (IGRAs) are a widely used immunodiagnostic method for TB. It has been proved to have an application value in the detection of *M. tuberculosis* infection, and is significantly superior to TST (tuberculin skin test). However, neither of IGRAs and TST can distinguish latent tuberculosis infection (LTBI) from active tuberculosis. In countries with high burdens of tuberculosis, the application of IGRAs is restricted due to a high proportion of latently infected people, and IGRAs can only be used as an auxiliary diagnostic tool. The tuberculosis diagnosis commonly used in clinic still mainly depends on clinical symptoms, imaging diagnosis and etiological diagnosis. If tuberculosis can be diagnosed accurately and quickly in the early stage and appropriate chemotherapy is performed, it will be of great value to reduction of the harm of TB patients and control of TB infection.

Therefore, there is need in this field to develop a method with high accuracy and specificity for diagnosing active tuberculosis simply, conveniently and quickly.

### Contents of Invention

In the invention, unless otherwise specified, the scientific and technical terms used herein have the meanings as generally understood by a person skilled in the art. Moreover, the laboratory operations of cell culture, biochemistry, nucleic acid chemistry and immunology used herein are the routine operations widely used in the corresponding fields. Meanwhile, in order to better understand the invention, the definitions and explanations of the relevant terms are provided as follows.

As used herein, the term "RV0183" or "RV0183 protein" refers to a protein naturally occurring in *M. tuberculosis,* which belongs to monoacylglycerol lipase (MAGL) or lysophospholipase. The sequence of RV0183 protein is well known in the art, and can be found in various public databases (for example, Accession No. CP009480.1, AIR12906.1, CCP42909.1, NP_214697.2, or WP_003401112.1 in NCBI database).

In the invention, when the amino acid sequence of RV0183 is mentioned, it is described by reference to the sequence as set forth in SEQ ID NO: 1. However, a person skilled in the art understands that *M. tuberculosis* includes not only the standard strain H37Rv, but also various isolated strains, and there might be difference in the amino acid sequence of RV0183 protein among various isolated strains. Furthermore, a person skilled in the art understands that although there might be difference in sequence, RV0183 proteins from different isolated strains of *M. tuberculosis,* have a very high identity in amino acid sequence, and have substantively the same biological function. In addition, a person skilled in the art understands that in the amino acid sequence of RV0183 protein, mutation or variation (including, but not limited to, substitution, deletion and/or addition) may occur naturally or be introduced artificially, without affecting the biological function thereof. Therefore, in the invention, the term "RV0183" includes not only the protein set forth in SEQ ID NO: 1, but also the RV0183 protein from various isolated strains of *M. tuberculosis* (for example, RV0183 protein set forth in BAL64025.1, AOE34503.1, EGE52872.1, or CDM08372.1), and the natural or artificial variants thereof. Moreover, when a sequence fragment of RV0183 protein is described, it includes not only a sequence fragment of SEQ ID NO: 1, but also the corresponding sequence fragment of the RV0183 protein from various isolated strains of *M. tuberculosis,* and the corresponding sequence fragment of the natural or artificial variants of SEQ ID NO: 1. Therefore, the expression "amino acid residues from positions 1 to 20 of RV0183 protein" includes the amino acid residues from positions 1 to 20 of SEQ ID NO: 1, the corresponding fragment of the RV0183 protein from various isolated strains of *M. tuberculosis,* and the corresponding fragment of the natural or artificial variants of RV0183 protein. In the invention, the expression "corresponding fragment" refers to the fragment that is located in equivalent positions of sequences when the sequences are subjected to optimized alignment, i.e. the sequences are aligned to obtain a highest percentage of identity.

In the invention, the term "RV0183" or "RV0183 protein" is not restricted by any particular method for synthesis of protein, and can be produced by conventional techniques known by a person skilled in the art, for example, recombinant DNA technique or chemical synthesis technique.

As used herein, the term "PlcD" or "PlcD protein" refers to a protein naturally occurring in *M. tuberculosis,* which belongs to phospholipase C family. The sequence of PlcD protein is well known in the art, see, for example, Andersen ST, et al., Nature, 1998. 393(6685): 537-44 (which is incorporated herein by reference), and Accession No. CCP44521.1 in NCBI database.

In the invention, when the amino acid sequence of PlcD is mentioned, it is described by reference to the sequence as set forth in SEQ ID NO: 3. However, a person skilled in the art understands that *M. tuberculosis* includes not only the standard strain H37Rv, but also various isolated strains, and there might be difference in the amino acid sequence of PlcD protein among various isolated strains. Furthermore, a person skilled in the art understands that although there might be difference in sequence, PlcD proteins from different isolated strains of *M. tuberculosis,* have a very high identity in amino acid sequence, and have substantively the same biological function. In addition, a person skilled in the art understands that in the amino acid sequence of PlcD protein, mutation or variation (including, but not limited to, substitution, deletion and/or addition) may occur naturally or be introduced artificially, without affecting the biological function thereof. Therefore, in the invention, the term "PlcD" includes not only the protein set forth in SEQ ID NO: 3, but also the PlcD protein from various isolated strains of *M. tuberculosis,* and its natural or artificial variants. Moreover, when a sequence fragment of PlcD protein is described, it includes not only a sequence fragment of SEQ ID NO: 3, but also the corresponding sequence fragment of the PlcD protein from various isolated strains of *M. tuberculosis,* and the corresponding sequence fragment of the natural or artificial variants of SEQ ID NO: 3. In the invention, the expression "corresponding fragment" refers to the fragment that is located in equivalent positions of sequences when the sequences are subjected to optimized alignment, i.e. the sequences are aligned to obtain a highest percentage of identity.

In the invention, the term "PlcD" or "PlcD protein" is not restricted by any particular method for synthesis of protein, and can be produced by conventional techniques known by a person skilled in the art, for example, recombinant DNA technique or chemical synthesis technique.

As used herein, the expression "antigenic fragment of RV0183 or PlcD" refers to the amino acid sequence fragment (i.e. polypeptide) obtained by the truncation of RV0183 or PlcD protein, which has the same biological activity as the corresponding full-length protein, i.e. capability of stimulating peripheral blood from aTB patients to generate IL-6, so as to distinguish an aTB population from a non-aTB population (for example, individuals with latent tuberculosis infection, patients with old tuberculosis or individuals without tuberculosis infection). For example, in the invention, the amino acid sequence fragments set forth in SEQ ID NOs: 5-25 are antigenic fragments of RV0183. In the invention, the antigenic fragments are not restricted by any particular method for synthesis of polypeptide, and can be produced by conventional techniques known by a person skilled in the art, for example, recombinant DNA technique or chemical synthesis technique.

In the invention, RV0183, PlcD, or antigenic fragments thereof (i.e. polypeptides) can be obtained by recombinant DNA technique, for example, obtained from polynucleotides encoding these proteins or polypeptides by using a cell-free expression system (a cell-free expression system includes, for example, a reticulocyte lysate-based expression system, a wheat germ extract-based expression system, and an *E. coli* extract-based expression system); or obtained from polynucleotides encoding these proteins or polypeptides by using an *in vivo* expression system (for example, an *E. coli* prokaryotic expression system, a yeast eukaryotic expression system). As an alternative, RV0183, PlcD, or antigenic fragments thereof (i.e. polypeptides) can be produced by chemical synthesis. Methods for chemical total synthesis of proteins or polypeptides are well known in the art (see, for example, Raibaut L, et al., Top Curr Chem. 2015; 363: 103-54; Thapa P, et al. Molecules. 2014; 19(9):14461-83; Dawson PE, et al., Science, 1994; 266(5186): 776-9; and Wang P, et al., Tetrahedron Lett, 1998, 39(47): 88711-14; which is incorporated herein by reference), and including, but not limited to: Solid Phase Peptide Synthesis (SPPS) or Liquid Phase Segment Ligation Synthesis (for example, Native Chemical Ligation, NCL, Azide method, Transfer Active Ester Condensation (TAEC)).

As used herein, the expression "antigenic fragments having the amino acid sequences set forth in SEQ ID NOs: 13, 14 and 19" refers to the combination of the antigenic fragment having an amino acid sequence set forth in SEQ ID NO: 13, the antigenic fragment having an amino acid sequence set forth in SEQ ID NO: 14, and the antigenic fragment having an amino acid sequence set forth in SEQ ID NO: 19. Other similar expressions have similar meanings.

As used herein, the term "a reagent capable of detecting IL-6" refers to a substance that can specifically bind to IL-6. Such substances are well known in the art, or can be prepared by methods well known in the art, for example, an antibody, a targeting polypeptide or an aptamer, etc. In general, it is particularly preferred that such reagents can determine the level of IL-6 in a sample by immunologic assay. The use of immunologic assay is particularly advantageous as it utilizes the specific interaction/binding affinity between an antigen and an antibody. Therefore, as long as a reagent retains the reactivity of specifically binding to IL-6, the reagent can determine the level of IL-6 in a sample by immunologic assay (that is, the reagent can be used as a reagent capable of detecting IL-6). Various reagents, which retain the reactivity of specifically binding to IL-6, can be readily envisaged and can be obtained easily by a person skilled in the art, and including, but not limited to, an anti-IL-6 antibody or an antigen binding fragment thereof, for example, a polyclonal or monoclonal antibody against IL-6, such as an IgG antibody or an IgM antibody. As an alternative, the reagent capable of detecting IL-6 can also be an aptamer that binds to IL-6, including, but not limited to the aptamers described in PCT international application WO2014159669 and "Gupta S, et al., J Biol Chem. 2014; 289(12): 8706-19", which are incorporated herein by reference.

As used herein, the term "specific binding/specifically bind" refers to the non-random binding reaction between two molecules (i.e. a binding molecule and a target molecule), for example, the reaction between an antibody and an antigen that the antibody is directed to. The binding affinity between two molecules can be described by K_{D} value. K_{D} value refers to a dissociation constant, which is a ratio of kd (a dissociation rate of a specific binding molecule-target molecule interaction; also called koff) to ka (an association rate of a specific binding molecule-target molecule interaction; also called kon); or refers to kd/ka which is expressed as molar concentration (M). The lower the K_{D} value is, the tighter the binding of the two molecules is, and the higher the affinity is. In some embodiments, an antibody specifically binding to a certain antigen (or an antibody specific for a certain antigen) means that the antibody binds to the antigen with an affinity (K_{D}) of below about 10⁻⁵ M, for example, below about 10⁻⁶ M, 10⁻⁷ M, 10⁻⁸ M, 10⁻⁹ M or 10⁻¹⁰ M or less. K_{D} value can be determined by methods well known in the art, for example, by surface plasmon resonance (SPR) in BIACORE instrument.

As used herein, the term "immunologic assay" refers to an assay utilizing the specific interaction/binding affinity between an antigen and an antibody, which is generally used to determine the presence or level of a specific antigen or antibody in a sample. Such immunologic assays are well known for a person skilled in the art, including, but not limited to ELISA assay, Elispot assay, Western blot, surface plasmon resonance, and the like. The detailed description of immunologic assay, can be found in, for example, Fundamental Immunology, Ch. 7 Paul, W., ed., the 2nd edition, Raven Press, N.Y. (1989).

As used herein, the term "antibody" generally refers to an immunoglobulin molecule consisting of two pairs of polypeptide chains (each has a light (L) chain and a heavy (H) chain). Light chain of an antibody may be classified into κ and λ light chain. Heavy chain may be classified into µ, δ, γ, α and ε, which define isotypes of an antibody as IgM, IgD, IgG, IgA and IgE, respectively. In a light chain and a heavy chain, a variable region is linked to a constant region via a "J" region of about 12 or more amino acids, and a heavy chain further comprises a "D" region of about 3 or more amino acids. Each heavy chain consists of a heavy chain variable region (VH) and a heavy chain constant region (CH). A heavy chain constant region consists of 3 domains (CH1, CH2 and CH3). Each light chain consists of a light chain variable region (VL) and a light chain constant region (CL). A light chain constant region consists of a domain CL. The constant region of an antibody can mediate the binding of an immunoglobulin to a host tissue or factor, including various cells (e.g., effector cells) of an immune system and the first component (C1q) of classical complement system. VH and VL regions can also be divided into hypervariable regions (called complementary determining regions (CDR)), which are interspaced by relatively conservative regions (called framework region (FR)). Each VH and VL consists of 3 CDRs and 4 FRs in the following order: FR1, CDR1, FR2, CDR2, FR3, CDR3, FR4 from N-terminal to C-terminal. The variable region (VH and VL) of each heavy/light chain pair forms an antigen binding site, respectively. Distribution of amino acids in each region or domain follows the definition in Kabat Sequences of Proteins of Immunological Interest (National Institutes of Health, Bethesda, Md. (1987 and 1991)), or Chothia & Lesk (1987) J. Mol. Biol. 196:901-917; Chothia et al., (1989) Nature 342:878-883. The term "antibody" is not restricted by any specific method for producing antibodies. For example, antibodies include particularly, recombinant antibodies, monoclonal antibodies and polyclonal antibodies. Antibodies may be of different antibody isotypes, for example, IgG (e.g., IgG1, IgG2, IgG3 or IgG4 subtype), IgA1, IgA2, IgD, IgE or IgM antibody.

As used herein, "an antigen binding fragment" of an antibody refers to one or more parts of a full-length antibody, which retain the ability of binding to the same antigen (e.g. IL-6) that a full-length antibody binds to, and compete with a full-length antibody for specifically binding to the antigen. Generally, see Fundamental Immunology, Ch. 7 (Paul, W., ed., the second edition, Raven Press, N.Y. (1989), which is incorporated herein by reference for all purposes. Antigen binding fragments may be produced by recombinant DNA techniques or by enzymatic or chemical cleavage of an intact antibody. Under some conditions, antigen binding fragments include Fab, Fab', F(ab')₂, Fd, Fv, dAb and complementary determining region (CDR) fragments, single chain antibody (e.g. scFv), chimeric antibody, diabody and such polypeptides that comprise at least one part of an antibody sufficient to confer the specific antigen binding ability on the polypeptides.

As used herein, the term "Aptamer" refers to a single-stranded oligonucleotide that can bind to a target protein or other biological target molecule with high affinity and high specificity, which can be folded into a thermodynamically stable 3D structure such as Stem-Loop, Hairpin, Pseudoknot or G-tetramer, and specifically bind to a target protein or other biological target molecule, for example, via formation of complementary structure, base stacking force, van der Waals force, hydrogen bond or electrostatic interaction. An aptamer may be DNA or RNA, and may also include nucleic acid analogs (for example, locked nucleic acid (LNA), peptide nucleic acid (PNA), glycol nucleic acid (GNA) or threose nucleic acid (TNA)). Methods for obtaining an aptamer binding to a specific target protein are well known in the art, for example, SELEX (Systematic evolution of ligands by exponential enrichment) screening technology.

As used herein, the term "a targeting polypeptide" refers to a polypeptide molecule that can specifically bind to a target protein. In the invention, the targeting polypeptide may include naturally occurring amino acids, synthetic amino acids, or amino acid mimetics that work in a similar manner as naturally occurring amino acids do. Naturally occurring amino acids are amino acids that are encoded by genetic codes and those that are modified later, for example, hydroxyproline, γ-hydroxyglutamate, O-phospho-serine, phosphothreonine or phosphotyrosine. In the invention, the "specificity" between a targeting polypeptide and its target protein can be determined based on affinity, and the affinity can be described by a dissociation equilibrium constant (i.e. K_{D} value) between a targeting polypeptide and a target protein binding thereto. The lower the K_{D} value is, the tighter the binding between a targeting polypeptide and a target protein binding thereto is. It is well known in the art that a K_{D} value of above about 10⁻³ M is generally regarded as no binding or a non-specific binding. Based on a particular target protein, a targeting polypeptide specifically binding to the target protein can be obtained by methods known by a person skilled in the art, for example, screening by phage display technology or protein microarray technology.

As used herein, the term "a detectable label" refers to any composition that can be detected by fluorescent, spectral, photochemical, biochemical, immunological, electrical, optical or chemical means. In the invention, it is particularly preferred that such labels can be applied to immunologic assay (for example, enzyme linked immunosorbent assay, radioimmunoassay, fluorescent immunoassay, chemiluminescent immunoassay, etc.). Such labels are well known in the art, including, but not limited to, enzymes (for example, horseradish peroxidase, alkaline phosphatase, β-galactosidase, urease, glucose oxidase, etc.), radionuclide (for example, ³H, ¹²⁵I, ³⁵S, ¹⁴C or ³²P), fluorescent dyes (for example, fluorescein isothiocyanate (FITC), fluorescein, tetramethylrhodamine isothiocyanate (TRITC), phycoerythrin (PE), Texas Red, rhodamine, quantum dot or cyanine dye derivatives (e.g. Cy7, Alexa 750)), acridinium ester compounds, magnetic beads (e.g. Dynabeads®), calorimetric labels such as colloidal gold or colored glass or plastic (e.g. polystyrene, polypropylene, latex, etc.) beads, and biotin for binding to avidin (e.g. streptavidin) with which said labels are modified. The patents, which teach the use of the labels, include, but are not limited to US patents 3,817,837; 3,850,752; 3,939,350; 3,996,345; 4,277,437; 4,275,149; and 4,366,241 (all of which are incorporated herein by reference). The labels covered in the invention can be detected by methods known in the art. For example, radiolabels can be detected using photographic film or scintillation counters, and fluorescent labels can be detected by determining the emitted light using a photodetector. Enzyme labels are generally detected by providing a substrate for the enzyme and detecting the reaction product resulted from the reaction between the enzyme and the substrate, and calorimetric labels are detected by simple visualization of the stained labels.

As used herein, the term "a specific stimulating agent" refers to a substance that can only stimulate PBMC from aTB patients to generate IL-6, but cannot stimulate PBMC from a non-aTB population (for example, individuals with latent tuberculosis infection (LTBI), patients with old tuberculosis or individuals without tuberculosis infection (tested negative in IGRA)) to generate IL-6; or refers to such a substance that stimulates PBMC from aTB patients to generate IL-6 in an amount significantly higher than PBMC from non-aTB populations (for example, individuals with latent tuberculosis infection (LTBI), patients with old tuberculosis or individuals without tuberculosis infection (tested negative in IGRA)). The specific stimulating agent according to the invention includes RV0183, PlcD, or antigenic fragments thereof. In the preferred embodiments of the invention, the specific stimulating agent contains no or almost no endotoxin. Methods for removing endotoxin from said specific stimulating agent are well known in the art, for example, ion exchange chromatography, affinity chromatography or extraction.

As used herein, the term "a non-specific stimulating agent" refers to a substance that can stimulate most or all of lymphocytes, without being restricted by TCR or BCR specificity. Activated lymphocytes can secrete a lot of cytokines (e.g. IL-6). Substances that can be used as non-specific stimulating agents, are well known in the art, including, but not limited to mitogen, phytohemagglutinin (PHA), Concanavalin A (ConA), pokeweed mitogen (PWM), lipopolysaccharide (LPS), or staphylococcal protein A (SPA). In some embodiments, the non-specific stimulating agent may be comprised in a culture medium (for example, a cell culture medium, such as RPMI-1640 medium and DMEM medium).

As used herein, the term "a statistical analysis value" refers to a value obtained by statistical analysis of the results obtained by various detection methods. Various statistical analysis methods are well known in the art (see, for example, PCT international application WO2009064901), and include, but are not limited to Linear combination, Linear regression model, Logistic regression model, Linear discrimination analysis (LDA) model, The nearest neighbor model and Prediction Analysis of Microarrays (PAM) of the detection results. In general, it is particularly preferred that the statistical analysis value is a value obtained by statistical analysis using Logistic regression model. Logistic regression model is particularly described in, for example, "Hu Chunyan. Serum level of four tumor markers in women with ovarian cancer [D]. Guangzhou: Sun Yat-sen University, 2008: 1-39".

As used herein, the term "a reference value" (also called the optimal diagnostic critical value) refers to a value that can reflect the status of a non-aTB population. In the invention, a reference value includes, for example, a normal value or range determined depending on a difference value in the level of IL-6 of samples from a non-aTB population (for example, individuals with latent tuberculosis infection (LTBI), patients with old tuberculosis or individuals without tuberculosis infection (tested negative in IGRA)) before and after the stimulation with a specific stimulating agent; and a value (a statistical analysis value) obtained by statistical analysis of the determined values (for example, the difference value in the level of IL-6 as described above) obtained from samples of a non-aTB population. Methods for determining an optimal diagnostic critical value are well known in the art, including, but not limited to Receiver Operating Characteristic (ROC) curve analysis, which is particularly described in, for example, "Habibzadeh F, et al., Biochem Med (Zagreb). 2016; 26(3):297-307" and "Chen Weizhong et al., Selection of optimal operating point for ROC curve [J]. Chinese Journal of Health Statistics, 2006, 23:157-158".

As used herein, the term "Peripheral blood mononuclear cell (PBMC)" is a generic name for cells having a single nucleus in peripheral blood, including, but not limited to lymphocytes (T cells, B cells, NK cells) and monocytes or dendritic cells. Methods for obtaining PBMC from peripheral blood are well known in the art, including, but not limited to Ficoll method or Percoll method.

As used herein, the term "peripheral blood buffy coat" refers to a component formed after natural sedimentation, centrifugation or density gradient centrifugation of peripheral anticoagulant blood, and consisting mainly of white blood cells (including peripheral blood mononuclear cells) and platelets. After centrifugation of anticoagulant blood, there are an upper layer of plasma, a bottom layer of red blood cells, and a white thin layer (called buffy coat) between them, which accounts for about 1% of total blood by volume.

As used herein, the term "subject" includes, but is not limited to, various animal, particularly mammal, for example, human.

As used herein, the term "an anticoagulant" refers to an agent or a substance that can prevent blood coagulation. Such substances are well known in the art, including, but not limited to heparin, EDTA, oxalates (e.g. sodium oxalate, potassium oxalate, ammonium oxalate), and sodium citrate.

In the invention, the term "a diluent" is preferably an electrolyte solution that can retain the cell osmotic pressure, and if necessary, can also act to retain the physiological pH value. Such solutions are well known in the art, including, but not limited to Alsever's solution, Earle's Balanced Salt Solution (EBSS), Gey's Balanced Salt Solution (GBSS), Hanks' Balanced Salt Solution (HBSS), Phosphate Buffered Saline (PBS), Dulbecco's Phosphate Buffered Saline (DPBS), Puck's Balanced Salt Solution, Ringer's Balanced Salt Solution (RBSS), Simm's Balanced Salt Solution (SBSS), TRIS-buffered saline (TBS), Tyrode's Balanced Salt Solution (TBSS), physiological saline or Ringer's Solution.

As used herein, the term "a culture solution" or "a culture medium" refers to nutrients capable of maintaining the viability of cells. Usually, the nutrients contain amino acids, vitamins, carbohydrates, inorganic salts, etc. Such nutrients are well known in the art, including, but not limited to RPMI-1640 medium or DMEM medium. In the invention, the purpose of adding a culture solution or a culture medium into a sample from a subject is to maintain the viability of cells, especially PBMC in the sample, during the stimulation with a stimulating agent. Methods for maintaining the viability of cells in a blood component are well known in the art, which can be selected by a person skilled in the art depending on practical needs. In some embodiments, when the sample is whole blood, a culture solution can be added, wherein the culture solution may be, for example, phosphate buffer or physiological saline, to which an appropriate amount of glucose, sodium chloride, potassium chloride, and the like are added. In some embodiments, the culture solution is phosphate buffer, to which an appropriate amount of glucose and potassium chloride are added. In some embodiments, when the sample is peripheral blood mononuclear cell (PBMC), peripheral blood buffy coat or other blood components containing PBMC, a culture medium, for example, a cell culture medium (e.g. a cell culture medium suitable for maintaining the viability of blood cells, especially PBMC, such as RPMI-1640 medium or DMEM medium), can be added.

As used herein, the term "active tuberculosis (aTB)" means that the focus of tuberculosis is active, for example, tested positive in sputum smear test, or accompanied by related symptoms such as low fever, cough, weight loss, fatigue, poor appetite, and so on. In the invention, tuberculosis includes pulmonary tuberculosis and extrapulmonary tuberculosis. The extrapulmonary tuberculosis includes, for example, lymphatic tuberculosis, tuberculous meningitis, tuberculous peritonitis, intestinal tuberculosis, renal tuberculosis, epididymal tuberculosis, tuberculosis of female reproductive system (including fallopian tube, endometrial, ovarian tuberculosis), bone and joint tuberculosis, etc.

The inventors of the present application screened a large number of antigens of RD region of *M. tuberculosis*, and surprisingly found that RV0183, PlcD, or antigenic fragments thereof can stimulate peripheral blood from aTB patients to generate a large amount of IL-6, and therefore can distinguish an aTB population from a non-aTB population (for example, individuals with latent tuberculosis infection (LTBI), patients with old tuberculosis or individuals without tuberculosis infection). However, before the present application, the IGRAs and TST commonly used in the art cannot distinguish latent tuberculosis infection (LTBI) from active tuberculosis. Based on this discovery, the inventors develop a new method for diagnosing active tuberculosis.

Therefore, in one aspect, the invention provides a kit comprising one or more of RV0183, PlcD, or antigenic fragments thereof, and a reagent capable of detecting IL-6.

In some preferred embodiments, the RV0183 has an amino acid sequence set forth in SEQ ID NO: 1; and/or, the PlcD has an amino acid sequence set forth in SEQ ID NO: 3.

In some preferred embodiments, the kit comprises RV0183 and/or PlcD.

In some preferred embodiments, the kit comprises one or more antigenic fragments of RV0183. In some further preferred embodiments, the antigenic fragments have the amino acid sequences selected from: SEQ ID NOs: 5-25.

In some preferred embodiments, the kit comprises antigenic fragments having the amino acid sequences set forth in SEQ ID NOs: 13, 14 and 19.

Optionally, the kit further comprises a combination of the following antigenic fragments:
1) antigenic fragments having the amino acid sequences set forth in SEQ ID NOs: 5, 11 and 22,
2) antigenic fragments having the amino acid sequences set forth in SEQ ID NOs: 7-8 and 11-12,
3) antigenic fragments having the amino acid sequences set forth in SEQ ID NOs: 5-7, 11-12, 22 and 24, or
4) antigenic fragments having the amino acid sequences set forth in SEQ ID NOs: 5, 8-10, 12, 15 and 22-25.

In some preferred embodiments, the kit comprises antigenic fragments having the amino acid sequences set forth in SEQ ID NOs: 5-25.

In some preferred embodiments, the reagent capable of detecting IL-6 is a substance that can specifically bind to IL-6, for example, an antibody, a targeting polypeptide or an aptamer.

Optionally, the reagent further comprises a detectable label.

In some preferred embodiments, the reagent determines the level of IL-6 in the sample by immunologic assay. In some further preferred embodiments, the immunologic assay is selected from the group consisting of ELISA assay, Elispot assay, Western blot, and surface plasmon resonance.

In some preferred embodiments, the reagent comprises an anti-IL-6 antibody or an antigen binding fragment thereof. In some further preferred embodiments, the reagent determines the level of IL-6 by ELISA.

In some preferred embodiments, the anti-IL-6 antibody is a monoclonal antibody or a polyclonal antibody. In some further preferred embodiments, the anti-IL-6 antibody is an IgG antibody or an IgM antibody.

In some preferred embodiments, the kit further comprises one or more devices or reagents selected from 1) to 5):
1) a blood collection device, e.g. a pyrogen-free vacuum blood collection tube;
2) an anticoagulant, e.g. heparin;
3) a culture solution or a culture medium;
4) a non-specific stimulating agent, e.g. phytohemagglutinin or Concanavalin A;
5) a diluent, e.g. phosphate buffer or physiological saline.

In some preferred embodiments, the kit is useful for diagnosing active tuberculosis, determining the therapeutic effect of a therapy on active tuberculosis or screening a candidate drug capable of treating active tuberculosis.

In another aspect, the invention provides use of a specific stimulating agent in the manufacture of a kit for diagnosing active tuberculosis; wherein, the specific stimulating agent is one or more selected from the group consisting of RV0183, PlcD, and antigenic fragments thereof.

In some preferred embodiments, the RV0183 has an amino acid sequence set forth in SEQ ID NO: 1; and/or, the PlcD has an amino acid sequence set forth in SEQ ID NO: 3.

In some preferred embodiments, the specific stimulating agent is selected from the group consisting of RV0183, PlcD, and a combination thereof.

In some preferred embodiments, the specific stimulating agent is selected from one or more antigenic fragments of RV0183. In some further preferred embodiments, the antigenic fragments have the amino acid sequences selected from: SEQ ID NOs: 5-25.

In some preferred embodiments, the specific stimulating agent comprises antigenic fragments having the amino acid sequences set forth in SEQ ID NOs: 13, 14 and 19.

Optionally, the specific stimulating agent further comprises a combination of the following antigenic fragments:
1) antigenic fragments having the amino acid sequences set forth in SEQ ID NOs: 5, 11 and 22,
2) antigenic fragments having the amino acid sequences set forth in SEQ ID NOs: 7-8 and 11-12,
3) antigenic fragments having the amino acid sequences set forth in SEQ ID NOs: 5-7, 11-12, 22 and 24, or
4) antigenic fragments having the amino acid sequences set forth in SEQ ID NOs: 5, 8-10, 12, 15 and 22-25.

In some preferred embodiments, the specific stimulating agent comprises antigenic fragments having the amino acid sequences set forth in SEQ ID NOs: 5-25.

In some preferred embodiments, the kit comprises a reagent capable of detecting IL-6, such as an antibody, a targeting polypeptide or an aptamer that can specifically bind to IL-6.

Optionally, the reagent further comprises a detectable label.

In some preferred embodiments, the reagent determines the level of IL-6 in the sample by immunologic assay. In some further preferred embodiments, the immunologic assay is selected from the group consisting of ELISA assay, Elispot assay, Western blot, and surface plasmon resonance.

In some preferred embodiments, the reagent comprises an anti-IL-6 antibody or an antigen binding fragment thereof. In some further preferred embodiments, the reagent determines the level of IL-6 by ELISA.

In some preferred embodiments, the anti-IL-6 antibody is a monoclonal antibody or a polyclonal antibody. In some further preferred embodiments, the anti-IL-6 antibody is an IgG antibody or an IgM antibody.

In some preferred embodiments, the kit further comprises one or more devices or reagents selected from 1) to 5):
1) a blood collection device, e.g. a pyrogen-free vacuum blood collection tube;
2) an anticoagulant, e.g. heparin;
3) a culture solution or a culture medium;
4) a non-specific stimulating agent, e.g. phytohemagglutinin or Concanavalin A;
5) a diluent, e.g. phosphate buffer or physiological saline.

In some preferred embodiments, the kit diagnoses whether the subject is suffering from active tuberculosis, by a method comprising the following steps:
(1) stimulating at least one sample from the subject with a specific stimulating agent, and using the at least one sample as a test sample, and using a non-stimulated sample from the subject, as a negative control sample, wherein the specific stimulating agent is one or more selected from the group consisting of RV0183, PlcD, and antigenic fragments thereof;
(2) determining the level of IL-6 in each of the samples in the step (1) by using a reagent capable of detecting IL-6, and calculating a difference value in the level of IL-6 between the test sample and the negative control sample; and
(3) comparing the difference value with a reference value, or subjecting the difference value to statistical analysis so as to obtain a statistical analysis value, and comparing the statistical analysis value with a reference value, and determining whether the subject is suffering from active tuberculosis;
wherein, the sample comprises peripheral blood mononuclear cell (PBMC), for example, whole blood (e.g. anticoagulated whole blood), peripheral blood mononuclear cell (PBMC), or peripheral blood buffy coat, and optionally an additional component, for example, an anticoagulant, a diluent, etc.

In some preferred embodiments, when the difference value or the statistical analysis value obtained from the difference value is greater than the reference value, it indicates that the subject providing the sample is suffering from active tuberculosis; when the difference value or the statistical analysis value obtained from the difference value is not greater than the reference value, it indicates that the subject providing the sample is not suffering from active tuberculosis.

In some preferred embodiments, in the step (3), the difference value is subjected to statistical analysis by using a statistical model selected from the group consisting of: Linear combination, Linear regression model, Logistic regression model, Linear discrimination analysis (LDA) model, The nearest neighbor model and Prediction Analysis of Microarrays (PAM). In some further preferred embodiments, in the step (3), statistical analysis of the difference value is carried out by using Logistic regression model.

In some preferred embodiments, in the step (1), stimulating one or more samples from the subject with at least two specific stimulating agents together or separately, and using the one or more samples as the test sample, wherein the specific stimulating agents are each independently selected from the group consisting of RV0183, PlcD, and antigenic fragments thereof. In some further preferred embodiments, in the step (1), stimulating at least two samples with RV0183 and PlcD separately, and using the at least two samples as the test sample. Or, in some preferred embodiments, in the step (1), stimulating at least one sample with one or more antigenic fragments of RV0183 together, and using the at least one sample as the test sample.

In some preferred embodiments, in the step (1), the specific stimulating agent is contained in a culture medium, for example, a cell culture medium, such as RPMI-1640 medium or DMEM medium, and then is used to stimulate the sample from the subject, so as to generate a test sample.

In some preferred embodiments, the step (1) further comprises: stimulating at least one sample with a non-specific stimulating agent, and using the at least one sample as a positive control sample. In some further preferred embodiments, the non-specific stimulating agent includes phytohemagglutinin or Concanavalin A.

In some preferred embodiments, in the step (1), a sample from the subject is incubated or diluted with a culture medium that contains neither the specific stimulating agent nor a non-specific stimulating agent (for example, a cell culture medium, such as RPMI-1640 medium or DMEM medium), so as to generate a negative control sample.

In some preferred embodiments, prior to the step (1), the method further comprises one or more of the following steps: (a) obtaining the sample from the subject by using a blood collection device; (b) treating the blood collection device or the sample from the subject with an anticoagulant; (c) treating the sample from the subject with a culture solution or a culture medium; and, (d) diluting the sample from the subject with a diluent.

In some preferred embodiments, in the step (1), the sample from the subject is stimulated with the stimulating agent at a temperature (for example, 36-38 °C, e.g. about 37 °C) at which cells (such as PBMC) have a high activity.

In some preferred embodiments, in the step (1), the sample from the subject is stimulated with the stimulating agent for at least 12 h, for example, 15-24 h, e.g. 20-24 h.

In another aspect, the invention provides use of a specific stimulating agent in the manufacture of a kit for determining the therapeutic effect of a therapy on active tuberculosis; wherein, the specific stimulating agent is one or more selected from the group consisting of RV0183, PlcD, and antigenic fragments thereof.

In some preferred embodiments, the RV0183 has an amino acid sequence set forth in SEQ ID NO: 1; and/or, the PlcD has an amino acid sequence set forth in SEQ ID NO: 3.

In some preferred embodiments, the specific stimulating agent is selected from the group consisting of RV0183, PlcD, and a combination thereof.

In some preferred embodiments, the specific stimulating agent is selected from one or more antigenic fragments of RV0183. In some further preferred embodiments, the antigenic fragments have amino acid sequences selected from: SEQ ID NOs: 5-25.

In some preferred embodiments, the specific stimulating agent comprises antigenic fragments having the amino acid sequences set forth in SEQ ID NOs: 13, 14 and 19.

Optionally, the specific stimulating agent further comprises a combination of the following antigenic fragments:
1) antigenic fragments having the amino acid sequences set forth in SEQ ID NOs: 5, 11 and 22,
2) antigenic fragments having the amino acid sequences set forth in SEQ ID NOs: 7-8 and 11-12,
3) antigenic fragments having the amino acid sequences set forth in SEQ ID NOs: 5-7, 11-12, 22 and 24, or
4) antigenic fragments having the amino acid sequences set forth in SEQ ID NOs: 5, 8-10, 12, 15 and 22-25.

In some preferred embodiments, the specific stimulating agent comprises antigenic fragments having the amino acid sequences set forth in SEQ ID NOs: 5-25.

In some preferred embodiments, the kit comprises a reagent capable of detecting IL-6, such as an antibody, a targeting polypeptide or an aptamer that can specifically bind to IL-6.

Optionally, the reagent further comprises a detectable label.

In some preferred embodiments, the reagent determines the level of IL-6 in the sample by immunologic assay. In some further preferred embodiments, the immunologic assay is selected from the group consisting of ELISA assay, Elispot assay, Western blot, and surface plasmon resonance.

In some preferred embodiments, the reagent comprises an anti-IL-6 antibody or an antigen binding fragment thereof. In some further preferred embodiments, the reagent determines the level of IL-6 by ELISA.

In some preferred embodiments, the anti-IL-6 antibody is a monoclonal antibody or a polyclonal antibody. In some further preferred embodiments, the anti-IL-6 antibody is an IgG antibody or an IgM antibody.

In some preferred embodiments, the kit further comprises one or more devices or reagents selected from 1) to 5):
1) a blood collection device, e.g. a pyrogen-free vacuum blood collection tube;
2) an anticoagulant, e.g. heparin;
3) a culture solution or a culture medium;
4) a non-specific stimulating agent, e.g. phytohemagglutinin or Concanavalin A;
5) a diluent, e.g. phosphate buffer or physiological saline.

In some preferred embodiments, the kit determines the therapeutic effect of a therapy on active tuberculosis by a method comprising the following steps:
(1) before administering the therapy to a subject, obtaining at least two samples from the subject, as a pre-therapy sample;
(2) stimulating at least one pre-therapy sample from the subject with a specific stimulating agent, and using the at least one pre-therapy sample as a test sample, and using at least one non-stimulated pre-therapy sample from the subject, as a negative control sample, wherein the specific stimulating agent is one or more selected from the group consisting of RV0183, PlcD, and antigenic fragments thereof;
(3) determining the level of IL-6 in each of the samples in the step (2) by using a reagent capable of detecting IL-6, and calculating a difference value in the level of IL-6 between the test sample and the negative control sample, as a first difference value;
(4) administering the therapy to the subject;
(5) after administering the therapy to the subject, obtaining at least two samples from the subject, as a post-therapy sample;
(6) stimulating at least one post-therapy sample from the subject with a specific stimulating agent, and using the at least one post-therapy sample as a test sample, and using at least one non-stimulated post-therapy sample from the subject, as a negative control sample, wherein the specific stimulating agent is one or more selected from the group consisting of RV0183, PlcD, and antigenic fragments thereof;
(7) determining the level of IL-6 in each of the samples in the step (6) by using a reagent capable of detecting IL-6, and calculating a difference value in the level of IL-6 between the test sample and the negative control sample, as a second difference value; and
(8) comparing the second difference value with the first difference value, or separately subjecting the first difference value and the second difference value to statistical analysis so as to obtain a statistical analysis value of the first difference value and a statistical analysis value of the second difference value, and comparing the statistical analysis value of the second difference value with the statistical analysis value of the first difference value, and determining whether the therapy is effective in the treatment of active tuberculosis;
wherein, the sample comprises peripheral blood mononuclear cell (PBMC), for example, whole blood (e.g. anticoagulated whole blood), peripheral blood mononuclear cell (PBMC), or peripheral blood buffy coat, and optionally an additional component, for example, an anticoagulant, a diluent, etc.

In some preferred embodiments, when the second difference value is greater than the first difference value, or when the statistical analysis value of the second difference value is greater than the statistical analysis value of the first difference value, it indicates that the therapy is not effective in the treatment of active tuberculosis; when the second difference value is less than the first difference value, or when the statistical analysis value of the second difference value is less than the statistical analysis value of the first difference value, it indicates that the therapy is effective in the treatment of active tuberculosis.

In some preferred embodiments, in the step (8), statistical analysis of the first difference value and the second difference value is carried out by using a statistical model selected from the group consisting of: Linear combination, Linear regression model, Logistic regression model, Linear discrimination analysis (LDA) model, The nearest neighbor model and Prediction Analysis of Microarrays (PAM). In some further preferred embodiments, in the step (8), statistical analysis of the first difference value and the second difference value is carried out by using Logistic regression model.

In some preferred embodiments, in the steps (2) and (6), the pre-therapy sample and the post-therapy sample are subjected to the same treatment (for example, under the same conditions, treating with the same specific stimulating agent). In some preferred embodiments, in the steps (2) and (6), stimulating one or more samples from the subject with at least two specific stimulating agents together or separately, and using the one or more samples as the test sample, wherein the specific stimulating agents are each independently selected from the group consisting of RV0183, PlcD, and antigenic fragments thereof. In some further preferred embodiments, in the steps (2) and (6), stimulating at least two samples with RV0183 and PlcD separately, and using the at least two samples as the test sample. Or, in some preferred embodiments, in the steps (2) and (6), simulating at least one sample with one or more antigenic fragments of RV0183 together, and using the at least one sample as the test sample.

In some preferred embodiments, the subject is a mammal, such as human.

In some preferred embodiments, the therapy comprises administering an antitubercular agent to the subject, for example, isoniazid, rifampicin, streptomycin, pyrazinamide, ethambutol or any combination thereof.

In some preferred embodiments, in the steps (2) and (6), the specific stimulating agent is contained in a culture medium, for example, a cell culture medium, such as RPMI-1640 medium or DMEM medium, and then is used to stimulate the sample from the subject, so as to generate a test sample.

In some preferred embodiments, the steps (2) and (6) further comprise: stimulating at least one sample with a non-specific stimulating agent, and using the at least one sample as a positive control sample. In some further preferred embodiments, the non-specific stimulating agent includes phytohemagglutinin or Concanavalin A.

In some preferred embodiments, in the steps (2) and (6), a sample from the subject is incubated or diluted with a culture medium that contains neither the specific stimulating agent nor the non-specific stimulating agent (for example, a cell culture medium, such as RPMI-1640 medium or DMEM medium), so as to generate a negative control sample.

In some preferred embodiments, in the step (1), the pre-therapy sample from the subject is obtained by using a blood collection device.

In some preferred embodiments, in the step (5), the post-therapy sample from the subject is obtained by using a blood collection device.

In some preferred embodiments, prior to the step (1), the method further comprises one or more of the following steps: (a) treating the blood collection device or the sample from the subject with an anticoagulant; (b) treating the sample from the subject with a culture solution or a culture medium; and, (c) diluting the sample from the subject with a diluent.

In some preferred embodiments, prior to the step (5), the method further comprises one or more of the following steps: (a) treating the blood collection device or the sample from the subject with an anticoagulant; (b) treating the sample from the subject with a culture solution or a culture medium; and, (c) diluting the sample from the subject with a diluent.

In some preferred embodiments, in the steps (2) and (6), the sample from the subject is stimulated with the stimulating agent at a temperature (for example, 36-38 °C, e.g. about 37 °C) at which cells (such as PBMC) have a high activity.

In some preferred embodiments, in the steps (2) and (6), the sample from the subject is stimulated with the stimulating agent for at least 12 h, for example, 15-24 h, e.g. 20-24 h.

In another aspect, the invention provides use of a specific stimulating agent in the manufacture of a kit for screening a candidate drug capable of treating active tuberculosis; wherein, the specific stimulating agent is one or more selected from the group consisting of RV0183, PlcD, and antigenic fragments thereof.

In some preferred embodiments, the RV0183 has an amino acid sequence set forth in SEQ ID NO: 1; and/or, the PlcD has an amino acid sequence set forth in SEQ ID NO: 3.

In some preferred embodiments, the specific stimulating agent is selected from the group consisting of RV0183, PlcD, and a combination thereof.

In some preferred embodiments, the specific stimulating agent is selected from one or more antigenic fragments of RV0183. In some further preferred embodiments, the antigenic fragments have the amino acid sequences selected from: SEQ ID NOs: 5-25.

In some preferred embodiments, the specific stimulating agent comprises antigenic fragments having the amino acid sequences set forth in SEQ ID NOs: 13, 14 and 19.

Optionally, the specific stimulating agent further comprises a combination of the following antigenic fragments:
1) antigenic fragments having the amino acid sequences set forth in SEQ ID NOs: 5, 11 and 22,
2) antigenic fragments having the amino acid sequences set forth in SEQ ID NOs: 7-8 and 11-12,
3) antigenic fragments having the amino acid sequences set forth in SEQ ID NOs: 5-7, 11-12, 22 and 24, or
4) antigenic fragments having the amino acid sequences set forth in SEQ ID NOs: 5, 8-10, 12, 15 and 22-25.

In some preferred embodiments, the specific stimulating agent comprises antigenic fragments having the amino acid sequences set forth in SEQ ID NOs: 5-25.

In some preferred embodiments, the kit comprises a reagent capable of detecting IL-6, such as an antibody, a targeting polypeptide or an aptamer that can specifically bind to IL-6.

Optionally, the reagent further comprises a detectable label.

In some preferred embodiments, the reagent determines the level of IL-6 in the sample by immunologic assay. In some further preferred embodiments, the immunologic assay is selected from the group consisting of ELISA assay, Elispot assay, Western blot, and surface plasmon resonance.

In some preferred embodiments, the reagent comprises an anti-IL-6 antibody or an antigen binding fragment thereof. In some further preferred embodiments, the reagent determines the level of IL-6 by ELISA.

In some preferred embodiments, the anti-IL-6 antibody is a monoclonal antibody or a polyclonal antibody. In some further preferred embodiments, the anti-IL-6 antibody is an IgG antibody or an IgM antibody.

In some preferred embodiments, the kit further comprises one or more devices or reagents selected from 1) to 5):
1) a blood collection device, e.g. a pyrogen-free vacuum blood collection tube;
2) an anticoagulant, e.g. heparin;
3) a culture solution or a culture medium;
4) a non-specific stimulating agent, e.g. phytohemagglutinin or Concanavalin A;
5) a diluent, e.g. phosphate buffer or physiological saline.

In some preferred embodiments, the kit screens a candidate drug capable of treating active tuberculosis, by a method comprising the following steps:
(1) before administering the candidate drug to an model animal, obtaining at least two samples from the animal, as a pre-therapy sample;
(2) stimulating at least one pre-therapy sample from the animal with a specific stimulating agent, and using the at least one pre-therapy sample as a test sample, and using at least one non-stimulated pre-therapy sample from the animal, as a negative control sample, wherein the specific stimulating agent is one or more selected from the group consisting of RV0183, PlcD, and antigenic fragments thereof;
(3) determining the level of IL-6 in each of the samples in the step (2) by using a reagent capable of detecting IL-6, and calculating a difference value in the level of IL-6 between the test sample and the negative control sample, as a first difference value;
(4) administering athe candidate drug to the animal;
(5) after administering the candidate drug to the animal, obtaining at least two samples from the animal, as a post-therapy sample;
(6) stimulating at least one post-therapy sample from the animal with a specific stimulating agent, and using the at least one post-therapy sample as a test sample, and using at least one non-stimulated post-therapy sample from the animal, as a negative control sample, wherein the specific stimulating agent is one or more selected from the group consisting of RV0183, PlcD, and antigenic fragments thereof;
(7) determining the level of IL-6 in each of the samples in the step (6) by using a reagent capable of detecting IL-6, and calculating a difference value in level of IL-6 between the test sample and the negative control sample, as a second difference value; and
(8) comparing the second difference value with the first difference value, or separately subjecting the first difference value and the second difference value to statistical analysis so as to obtain a statistical analysis value of the first difference value and a statistical analysis value of the second difference value, and comparing the statistical analysis value of the second difference value with the statistical analysis value of the first difference value, and determining whether the therapy is effective in the treatment of active tuberculosis;
wherein, the sample comprises peripheral blood mononuclear cell (PBMC), for example, whole blood (e.g. anticoagulated whole blood), peripheral blood mononuclear cell (PBMC), or peripheral blood buffy coat, and optionally an additional component, for example, an anticoagulant, a diluent, etc.

In some preferred embodiments, when the second difference value is greater than the first difference value, or when the statistical analysis value of the second difference value is greater than the statistical analysis value of the first difference value, it indicates that the drug screened is not effective in the treatment of active tuberculosis; when the second difference value is less than the first difference value, or when the statistical analysis value of the second difference value is less than the statistical analysis value of the first difference value, it indicates that the drug screened is effective in the treatment of active tuberculosis.

In some preferred embodiments, in the step (8), statistical analysis of the first difference value and the second difference value is carried out by using a statistical model selected from the group consisting of: Linear combination, Linear regression model, Logistic regression model, Linear discrimination analysis (LDA) model, The nearest neighbor model and Prediction Analysis of Microarrays (PAM). In some further preferred embodiments, in the step (8), statistical analysis of the first difference value and the second difference value is carried out by using Logistic regression model.

In some preferred embodiments, in the steps (2) and (6), the pre-therapy sample and the post-therapy sample are subjected to the same treatment (for example, under the same conditions, treating with the same specific stimulating agent). In some preferred embodiments, in the steps (2) and (6), stimulating one or more samples from the animal with at least two specific stimulating agents together or separately, and using the one or more samples as the test sample, wherein the specific stimulating agents are each independently selected from the group consisting of RV0183, PlcD, and antigenic fragments thereof. In some further preferred embodiments, in the steps (2) and (6), stimulating at least two samples with RV0183 and PlcD separately, and using the at least two samples as the test sample. Or, in some preferred embodiments, in the steps (2) and (6), stimulating at least one sample with one or more antigenic fragments of RV0183 together, and using the at least one sample as the test sample.

In some preferred embodiments, the model animal is a non-human mammal, for example, a mouse, a guinea pig, a rabbit or a non-human primate (e.g. a cynomolgus monkey or a rhesus monkey).

In some preferred embodiments, the subject is a mammal, such as human.

In some preferred embodiments, in the steps (2) and (6), the specific stimulating agent is contained in a culture medium, for example, a cell culture medium, such as RPMI-1640 medium or DMEM medium, and then is used to stimulate the sample from the subject, so as to generate the test sample.

In some preferred embodiments, the steps (2) and (6) further comprise: stimulating at least one sample with a non-specific stimulating agent, and using the at least one sample as a positive control sample. In some further preferred embodiments, the non-specific stimulating agent includes phytohemagglutinin or Concanavalin A.

In some preferred embodiments, in the steps (2) and (6), the sample from the animal is incubated or diluted with a culture medium that contains neither the specific stimulating agent nor the non-specific stimulating agent (for example, a cell culture medium, such as RPMI-1640 medium or DMEM medium), so as to generate a negative control sample.

In some preferred embodiments, in the step (1), the pre-therapy sample from the animal is obtained by using a blood collection device.

In some preferred embodiments, in the step (5), the post-therapy sample from the animal is obtained by using a blood collection device.

In some preferred embodiments, prior to the step (1), the method further comprises one or more of the following steps: (a) treating the blood collection device or the sample from the animal with an anticoagulant; (b) treating the sample from the animal with a culture solution or a culture medium; and, (c) diluting the sample from the animal with a diluent.

In some preferred embodiments, prior to the step (5), the method further comprises one or more of the following steps: (a) treating the blood collection device or the sample from the animal with an anticoagulant; (b) treating the sample from the animal with a culture solution or a culture medium; and, (c) diluting the sample from the animal with a diluent.

In some preferred embodiments, in the steps (2) and (6), the sample from the animal is stimulated with the stimulating agent at a temperature (for example, 36-38 °C, e.g. about 37 °C) at which cells (such as PBMC) have a high activity.

In some preferred embodiments, in the steps (2) and (6), the sample from the animal is stimulated with the stimulating agent for at least 12 h, for example, 15-24 h, e.g. 20-24 h.

In another aspect, the invention provides a method for diagnosing whether a subject is suffering from active tuberculosis, comprising the following steps:
(1) providing at least two samples from the subject;
(2) stimulating at least one sample from the subject with a specific stimulating agent, and using the at least one sample as a test sample, and using a non-stimulated sample, as a negative control sample, wherein the specific stimulating agent is one or more selected from the group consisting of RV0183, PlcD, and antigenic fragments thereof;
(3) determining the level of IL-6 in each of the samples in the step (2), and calculating a difference value in the level of IL-6 between the test sample and the negative control sample; and
(4) comparing the difference value with a reference value, or subjecting the difference value to statistical analysis so as to obtain a statistical analysis value, and comparing the statistical analysis value with a reference value, and determining whether the subject is suffering from active tuberculosis;
wherein, the sample comprises peripheral blood mononuclear cell (PBMC), for example, whole blood (e.g. anticoagulated whole blood), peripheral blood mononuclear cell (PBMC), or peripheral blood buffy coat, and optionally an additional component, for example, an anticoagulant, a diluent, etc.

In some preferred embodiments, when the difference value or the statistical analysis value obtained from the difference value is greater than the reference value, it indicates that the subject providing the sample is suffering from active tuberculosis; when the difference value or the statistical analysis value obtained from the difference value is not greater than the reference value, it indicates that the subject providing the sample is not suffering from active tuberculosis.

In some preferred embodiments, in the step (4), the difference value is subjected to statistical analysis by using a statistical model selected from the group consisting of: Linear combination, Linear regression model, Logistic regression model, Linear discrimination analysis (LDA) model, The nearest neighbor model and Prediction Analysis of Microarrays (PAM). In some further preferred embodiments, in the step (4), statistical analysis of the difference value is carried out by using Logistic regression model.

In some preferred embodiments, the RV0183 has an amino acid sequence set forth in SEQ ID NO: 1; and/or, the PlcD has an amino acid sequence set forth in SEQ ID NO: 3.

In some preferred embodiments, the antigenic fragments are antigenic fragments of RV0183. In some further preferred embodiments, the antigenic fragments have the amino acid sequences selected from: SEQ ID NOs: 5-25.

In some preferred embodiments, the subject is a mammal, such as human.

In some preferred embodiments, in the step (2), stimulating one or more samples from the subject with at least two specific stimulating agents together or separately, and using the one or more samples as the test sample, wherein the specific stimulating agents are each independently selected from the group consisting of RV0183, PlcD, and antigenic fragments thereof.

In some preferred embodiments, in the step (2), stimulating at least two samples with RV0183 and PlcD separately, and using the at least two samples as the test sample. Or, in some preferred embodiments, in the step (2), stimulating at least one sample with one or more antigenic fragments together, and using the at least one sample as the test sample. In some further preferred embodiments, in the step (2), stimulating at least one sample with a combination of the following antigenic fragments together, and using the at least one sample as the test sample:
1) antigenic fragments having the amino acid sequences set forth in SEQ ID NOs: 13, 14 and 19,
2) antigenic fragments having the amino acid sequences set forth in SEQ ID NOs: 5, 11, 13-14, 19 and 22,
3) antigenic fragments having the amino acid sequences set forth in SEQ ID NOs: 7-8, 11-14 and 19,
4) antigenic fragments having the amino acid sequences set forth in SEQ ID NOs: 5-7, 11-14, 19, 22 and 24,
5) antigenic fragments having the amino acid sequences set forth in SEQ ID NOs: 5, 8-10, 12-15, 19 and 22-25; or
6) antigenic fragments having the amino acid sequences set forth in SEQ ID NOs: 5-25.

In some preferred embodiments, in the step (2), the specific stimulating agent is contained in a culture medium, for example, a cell culture medium, such as RPMI-1640 medium or DMEM medium, and then is used to stimulate the sample from the subject, so as to generate the test sample.

In some preferred embodiments, in the step (2), the sample from the subject is incubated or diluted with a culture medium that contains neither the specific stimulating agent nor the non-specific stimulating agent (for example, a cell culture medium, such as RPMI-1640 medium or DMEM medium), so as to generate a negative control sample.

In some preferred embodiments, in the step (3), the level of IL-6 in the samples is determined by immunologic assay. In some further preferred embodiments, the immunologic assay is selected from the group consisting of ELISA assay, Elispot assay, Western blot, and surface plasmon resonance.

In some preferred embodiments, in the step (3), the level of IL-6 is determined by using an anti-IL-6 antibody or an antigen binding fragment thereof, for example, by ELISA.

In some preferred embodiments, the anti-IL-6 antibody is a monoclonal antibody or a polyclonal antibody. In some further preferred embodiments, the anti-IL-6 antibody is an IgG antibody or an IgM antibody.

In some preferred embodiments, the step (2) further comprises: stimulating at least one sample with a non-specific stimulating agent, and using the at least one sample as a positive control sample. In some further preferred embodiments, the non-specific stimulating agent includes phytohemagglutinin or Concanavalin A.

In some preferred embodiments, prior to the step (1), the method further comprises one or more of the following steps: (a) obtaining the sample from the subject; (b) adding an anticoagulant such as heparin into the sample; (c) obtaining PBMC or a PBMC-containing blood component (e.g. peripheral blood buffy coat) from the sample; (d) adding a culture solution or a culture medium into the sample; (e) diluting the sample.

In some preferred embodiments, in the step (2), the sample from the subject is stimulated with the stimulating agent at a temperature (for example, 36-38 °C, e.g. about 37 °C) at which cells (such as PBMC) have a high activity.

In some preferred embodiments, in the step (2), the sample from the subject is stimulated with the stimulating agent for at least 12 h, for example, 15-24 h, e.g. 20-24 h.

In another aspect, the invention provides a method for determining the therapeutic effect of a therapy on active tuberculosis, comprising the following steps:
(1) before administering the therapy to a subject, obtaining at least two samples from the subject, as a pre-therapy sample;
(2) stimulating at least one pre-therapy sample from the subject with a specific stimulating agent, and using the at least one pre-therapy sample as a test sample, and using at least one non-stimulated pre-therapy sample from the subject, as a negative control sample, wherein the specific stimulating agent is one or more selected from the group consisting of RV0183, PlcD, and antigenic fragments thereof;
(3) determining the level of IL-6 in each of the samples in the step (2), and calculating a difference value in the level of IL-6 between the test sample and the negative control sample, as a first difference value;
(4) administering the therapy to the subject;
(5) after administering the therapy to the subject, obtaining at least two samples from the subject, as a post-therapy sample;
(6) stimulating at least one post-therapy sample from the subject with a specific stimulating agent, and using the at least one post-therapy sample as a test sample, and using at least one non-stimulated post-therapy sample from the subject, as a negative control sample, wherein the specific stimulating agent is one or more selected from the group consisting of RV0183, PlcD, and antigenic fragments thereof;
(7) determining the level of IL-6 in each of the samples in the step (6) by using a reagent capable of detecting IL-6, and calculating a difference value in the level of IL-6 between the test sample and the negative control sample, as a second difference value; and
(8) comparing the second difference value with the first difference value, or separately subjecting the first difference value and the second difference value to statistical analysis so as to obtain a statistical analysis value of the first difference value and a statistical analysis value of the second difference value, and comparing the statistical analysis value of the second difference value with the statistical analysis value of the first difference value, and determining whether the therapy is effective in the treatment of active tuberculosis;
wherein, the sample comprises peripheral blood mononuclear cell (PBMC), for example, whole blood (e.g. anticoagulated whole blood), peripheral blood mononuclear cell (PBMC), or peripheral blood buffy coat, and optionally an additional component, for example, an anticoagulant, a diluent, etc.

In some preferred embodiments, when the second difference value is greater than the first difference value, or when the statistical analysis value of the second difference value is greater than the statistical analysis value of the first difference value, it indicates that the therapy is not effective in the treatment of active tuberculosis; when the second difference value is less than the first difference value, or when the statistical analysis value of the second difference value is less than the statistical analysis value of the first difference value, it indicates that the therapy is effective in the treatment of active tuberculosis.

In some preferred embodiments, in the step (8), statistical analysis of the first difference value and the second difference value is carried out by using a statistical model selected from the group consisting of: Linear combination, Linear regression model, Logistic regression model, Linear discrimination analysis (LDA) model, The nearest neighbor model and Prediction Analysis of Microarrays (PAM). In some further preferred embodiments, in the step (8), statistical analysis of the first difference value and the second difference value is carried out by using Logistic regression model.

In some preferred embodiments, the RV0183 has an amino acid sequence set forth in SEQ ID NO: 1; and/or, the PlcD has an amino acid sequence set forth in SEQ ID NO: 3.

In some preferred embodiments, the antigenic fragments are antigenic fragments of RV0183. In some further preferred embodiments, the antigenic fragments have the amino acid sequences selected from: SEQ ID NOs: 5-25.

In some preferred embodiments, the subject is a mammal, such as human.

In some preferred embodiments, the therapy comprises administering an antitubercular agent to the subject, for example, isoniazid, rifampicin, streptomycin, pyrazinamide, ethambutol or any combination thereof.

In some preferred embodiments, in the steps (2) and (6), the pre-therapy sample and the post-therapy sample are subjected to the same treatment (for example, under the same conditions, treating with the same specific stimulating agent). In some preferred embodiments, in the steps (2) and (6), stimulating one or more samples from the subject with at least two specific stimulating agents together or separately, and using the one or more samples as the test sample, wherein the specific stimulating agents are independently selected from the group consisting of RV0183, PlcD, and antigenic fragments thereof.

In some preferred embodiments, in the steps (2) and (6), stimulating at least two samples with RV0183 and PlcD separately, and using the at least two samples as the test sample. Or, in some preferred embodiments, in the steps (2) and (6), stimulating at least one sample with one or more of the antigenic fragments together, and using the at least one sample as the test sample. In some further preferred embodiments, in the steps (2) and (6), stimulating at least one sample with a combination of the following antigenic fragments together, and using the at least one sample as the test sample:
1) antigenic fragments having the amino acid sequences set forth in SEQ ID NOs: 13, 14 and 19,
2) antigenic fragments having the amino acid sequences set forth in SEQ ID NOs: 5, 11, 13-14, 19 and 22,
3) antigenic fragments having the amino acid sequences set forth in SEQ ID NOs: 7-8, 11-14 and 19,
4) antigenic fragments having the amino acid sequences set forth in SEQ ID NOs: 5-7, 11-14, 19, 22 and 24,
5) antigenic fragments having the amino acid sequences set forth in SEQ ID NOs: 5, 8-10, 12-15, 19 and 22-25; or
6) antigenic fragments having the amino acid sequences set forth in SEQ ID NOs: 5-25.

In some preferred embodiments, in the steps (2) and (6), the specific stimulating agent is contained in a culture medium, for example, a cell culture medium, such as RPMI-1640 medium or DMEM medium, and then is used to stimulate the sample from the subject, so as to generate a test sample.

In some preferred embodiments, in the steps (2) and (6), the sample from the subject is incubated or diluted with a culture medium that contains neither the specific stimulating agent nor the non-specific stimulating agent (for example, a cell culture medium, such as RPMI-1640 medium or DMEM medium), so as to generate a negative control sample.

In some preferred embodiments, in the step (3), the level of IL-6 in the samples is determined by immunologic assay. In some further preferred embodiments, the immunologic assay is selected from the group consisting of ELISA assay, Elispot assay, Western blot, and surface plasmon resonance.

In some preferred embodiments, in the step (3), the level of IL-6 is determined by using an anti-IL-6 antibody or an antigen binding fragment thereof, for example, by ELISA.

In some preferred embodiments, the anti-IL-6 antibody is a monoclonal antibody or a polyclonal antibody. In some further preferred embodiments, the anti-IL-6 antibody is an IgG antibody or an IgM antibody.

In some preferred embodiments, the steps (2) and (6) further comprise: stimulating at least one sample with a non-specific stimulating agent, and using the at least one sample as a positive control sample. Further, the non-specific stimulating agent includes phytohemagglutinin or Concanavalin A.

In some preferred embodiments, prior to the step (1), the method further comprises one or more of the following steps: (a) adding an anticoagulant such as heparin into the pre-therapy sample; (b) obtaining PBMC or a PBMC-containing blood component (e.g. peripheral blood buffy coat) from the pre-therapy sample; (c) adding a culture solution or a culture medium into the pre-therapy sample; and, (d) diluting the pre-therapy sample.

In some preferred embodiments, prior to the step (5), the method further comprises one or more of the following steps: (a) adding an anticoagulant such as heparin into the post-therapy sample; (b) obtaining PBMC or a PBMC-containing blood component (e.g. peripheral blood buffy coat) from the post-therapy sample; (c) adding a culture solution or a culture medium into the post-therapy sample; and, (d) diluting the post-therapy sample.

In some preferred embodiments, in the steps (2) and (6), the sample from the subject is stimulated with the stimulating agent at a temperature (for example, 36-38 °C, e.g. about 37 °C) at which cells (such as PBMC) have a high activity.

In some preferred embodiments, in the steps (2) and (6), the sample from the subject is stimulated with the stimulating agent for at least 12 h, for example, 15-24 h, e.g. 20-24 h.

In another aspect, the invention provides a method for screening a candidate drug capable of treating active tuberculosis, comprising the following steps:
(1) before administering the candidate drug to an model animal, obtaining at least two samples from the animal, as a pre-therapy sample;
(2) stimulating at least one pre-therapy sample from the animal with a specific stimulating agent, and using the at least one pre-therapy sample as a test sample, and using at least one non-stimulated pre-therapy sample from the animal, as a negative control sample, wherein the specific stimulating agent is one or more selected from the group consisting of RV0183, PlcD, and antigenic fragments thereof;
(3) determining the level of IL-6 in each of the samples in the step (2), and calculating a difference value in the level of IL-6 between the test sample and the negative control sample, as a first difference value;
(4) administering the candidate drug to the animal;
(5) after administering the candidate drug to the animal, obtaining at least two samples from the animal, as a post-therapy sample;
(6) stimulating at least one post-therapy sample from the animal with a specific stimulating agent, and using the at least one post-therapy sample as a test sample, and using at least one non-stimulated post-therapy sample from the animal, as a negative control sample, wherein the specific stimulating agent is one or more selected from the group consisting of RV0183, PlcD, and antigenic fragments thereof;
(7) determining the level of IL-6 in each of the samples in the step (6) by using a reagent capable of detecting IL-6, and calculating a difference value in the level of IL-6 between the test sample and the negative control sample, as a second difference value; and
(8) comparing the second difference value with the first difference value, or separately subjecting the first difference value and the second difference value to statistical analysis so as to obtain a statistical analysis value of the first difference value and a statistical analysis value of the second difference value, and comparing the statistical analysis value of the second difference value with the statistical analysis value of the first difference value, and determining whether the therapy is effective in the treatment of active tuberculosis;
wherein, the sample comprises peripheral blood mononuclear cell (PBMC), for example, whole blood (e.g. anticoagulated whole blood), peripheral blood mononuclear cell (PBMC), or peripheral blood buffy coat, and optionally an additional component, for example, an anticoagulant, a diluent, etc.

In some preferred embodiments, when the second difference value is greater than the first difference value, or when the statistical analysis value of the second difference value is greater than the statistical analysis value of the first difference value, it indicates that the drug screened is not effective in the treatment of active tuberculosis; when the second difference value is less than the first difference value, or when the statistical analysis value of the second difference value is less than the statistical analysis value of the first difference value, it indicates that the drug screened is effective in the treatment of active tuberculosis.

In some preferred embodiments, in the step (8), statistical analysis of the first difference value and the second difference value is carried out by using a statistical model selected from the group consisting of: Linear combination, Linear regression model, Logistic regression model, Linear discrimination analysis (LDA) model, The nearest neighbor model and Prediction Analysis of Microarrays (PAM). In some further preferred embodiments, in the step (8), statistical analysis of the first difference value and the second difference value is carried out by using Logistic regression model.

In some preferred embodiments, the RV0183 has an amino acid sequence set forth in SEQ ID NO: 1; and/or, the PlcD has an amino acid sequence set forth in SEQ ID NO: 3.

In some preferred embodiments, the antigenic fragments are antigenic fragments of RV0183. In some further preferred embodiments, the antigenic fragments have the amino acid sequences selected from: SEQ ID NOs: 5-25.

In some preferred embodiments, the model animal is a non-human mammal, for example, a mouse, a guinea pig, a rabbit or a non-human primate (e.g. a cynomolgus monkey or a rhesus monkey).

In some preferred embodiments, the subject is a mammal, such as human.

In some preferred embodiments, in the steps (2) and (6), the pre-therapy sample and the post-therapy sample are subjected to the same treatment (for example, under the same conditions, treating with the same specific stimulating agent). In some preferred embodiments, in the steps (2) and (6), stimulating one or more samples from the animal with at least two specific stimulating agents together or separately, and using the one or more samples as the test sample, wherein the specific stimulating agents are each independently selected from the group consisting of RV0183, PlcD, and antigenic fragments thereof.

In some preferred embodiments, in the steps (2) and (6), stimulating at least two samples with RV0183 and PlcD separately, and using the at least two samples as the test sample. Or, in some preferred embodiments, in the steps (2) and (6), stimulating at least one sample with one or more of the antigenic fragments together, and using the at least one sample as the test sample. In some further preferred embodiments, in the steps (2) and (6), stimulating at least one sample with a combination of the following antigenic fragments together, and using the at least one sample as the test sample:
1) antigenic fragments having the amino acid sequences set forth in SEQ ID NOs: 13, 14 and 19,
2) antigenic fragments having the amino acid sequences set forth in SEQ ID NOs: 5, 11, 13-14, 19 and 22,
3) antigenic fragments having the amino acid sequences set forth in SEQ ID NOs: 7-8, 11-14 and 19,
4) antigenic fragments having the amino acid sequences set forth in SEQ ID NOs: 5-7, 11-14, 19, 22 and 24,
5) antigenic fragments having the amino acid sequences set forth in SEQ ID NOs: 5, 8-10, 12-15, 19 and 22-25; or
6) antigenic fragments having the amino acid sequences set forth in SEQ ID NOs: 5-25.

In some preferred embodiments, in the steps (2) and (6), the specific stimulating agent is contained in a culture medium, for example, a cell culture medium, such as RPMI-1640 medium or DMEM medium, and then is used to stimulate the sample from the subject, so as to generate a test sample.

In some preferred embodiments, in the steps (2) and (6), the sample from the animal is incubated or diluted with a culture medium that contains neither the specific stimulating agent nor the non-specific stimulating agent (for example, a cell culture medium, such as RPMI-1640 medium or DMEM medium), so as to generate a negative control sample.

In some preferred embodiments, in the step (3), the level of IL-6 in the samples is determined by immunologic assay. In some further preferred embodiments, the immunologic assay is selected from the group consisting of ELISA assay, Elispot assay, Western blot, and surface plasmon resonance.

In some preferred embodiments, in the step (3), the level of IL-6 is determined by using an anti-IL-6 antibody or an antigen binding fragment thereof, for example, by ELISA.

In some preferred embodiments, the anti-IL-6 antibody is a monoclonal antibody or a polyclonal antibody. In some further preferred embodiments, the anti-IL-6 antibody is an IgG antibody or an IgM antibody.

In some preferred embodiments, the steps (2) and (6) further comprise: stimulating at least one sample with a non-specific stimulating agent, and using the at least one sample as a positive control sample. In some further preferred embodiments, the non-specific stimulating agent includes phytohemagglutinin or Concanavalin A.

In some preferred embodiments, prior to the step (1), the method further comprises one or more of the following steps: (a) adding an anticoagulant such as heparin into the pre-therapy sample; (b) obtaining PBMC or a PBMC-containing blood component (e.g. peripheral blood buffy coat) from the pre-therapy sample; (c) adding a culture solution or a culture medium into the pre-therapy sample; and, (d) diluting the pre-therapy sample.

In some preferred embodiments, prior to the step (5), the method further comprises one or more of the following steps: (a) adding an anticoagulant such as heparin into the post-therapy sample; (b) obtaining PBMC or a PBMC-containing blood component (e.g. peripheral blood buffy coat) from the post-therapy sample; (c) adding a culture solution or a culture medium into the post-therapy sample; and, (d) diluting the post-therapy sample.

In some preferred embodiments, in the steps (2) and (6), the sample from the animal is stimulated with the stimulating agent at a temperature (for example, 36-38 °C, e.g. about 37 °C) at which cells (such as PBMC) have a high activity.

In some preferred embodiments, in the steps (2) and (6), the sample from the animal is stimulated with the stimulating agent for at least 12 h, for example, 15-24 h, e.g. 20-24 h.

In another aspect, the invention provides a polypeptide library, comprising:
a first peptide having an amino acid sequence set forth in SEQ ID NO: 13;
a second peptide having an amino acid sequence set forth in SEQ ID NO: 14; and
a third peptide having an amino acid sequence set forth in SEQ ID NO: 19.

Optionally, the polypeptide library further comprises a combination of the following polypeptides:
1) polypeptides having the amino acid sequences set forth in SEQ ID NOs: 5, 11 and 22,
2) polypeptides having the amino acid sequences set forth in SEQ ID NOs: 7-8 and 11-12,
3) polypeptides having the amino acid sequences set forth in SEQ ID NOs: 5-7, 11-12, 22 and 24, or
4) polypeptides having the amino acid sequences set forth in SEQ ID NOs: 5, 8-10, 12, 15 and 22-25.

In some preferred embodiments, the polypeptide library comprises antigenic fragments having the amino acid sequences set forth in SEQ ID NOs: 5-25.

In some preferred embodiments, the polypeptide library can induce the generation of IL-6 in a sample; wherein, the sample comprises peripheral blood mononuclear cell (PBMC), for example, whole blood (e.g. anticoagulated whole blood), peripheral blood mononuclear cell (PBMC), or peripheral blood buffy coat, and optionally an additional component, for example, an anticoagulant, a diluent, etc.

In some preferred embodiments, the polypeptide library is useful for diagnosing active tuberculosis, determining the therapeutic effect of a therapy on active tuberculosis or screening a candidate drug capable of treating active tuberculosis.

### Beneficial effects of the invention

It is discovered through a large number of trials and errors in the invention that a specific stimulating agent (RV0183, PlcD, or antigenic fragments thereof) can stimulate peripheral blood from aTB patients to generate a large amount of IL-6, and therefore can distinguish an aTB population from a non-aTB population (for example, individuals with latent tuberculosis infection (LTBI), patients with old tuberculosis or individuals without tuberculosis infection), thereby establishing a simple, convenient, and quick method for diagnosing active tuberculosis, which has high accuracy and specificity.

In some preferred embodiments, the stimulation of a sample from the subject can be accomplished by collecting whole blood, adding a specific stimulating agent, and then incubating under appropriate conditions. There are no high demands for experimental conditions, a person's technical ability, equipment and environment. As compared with traditional means for tuberculosis diagnosis (e.g. bacterial culture, sputum smear test, X-ray detection, etc.), it has higher sensitivity and specificity for the detection of aTB patients, and greatly reduces the time required for diagnosis; and the cost is not high, and it can be used widely.

The embodiments of the invention are described in detail by reference to the following drawings and examples. However, a person skilled in the art would understand that the following drawings and examples are used only for the purpose of illustrating the invention, rather than defining the scope of the invention. According to the detailed illustration of the following drawings and preferred embodiments, various purposes and advantages of the invention are apparent for a person skilled in the art.

### Description of the drawings

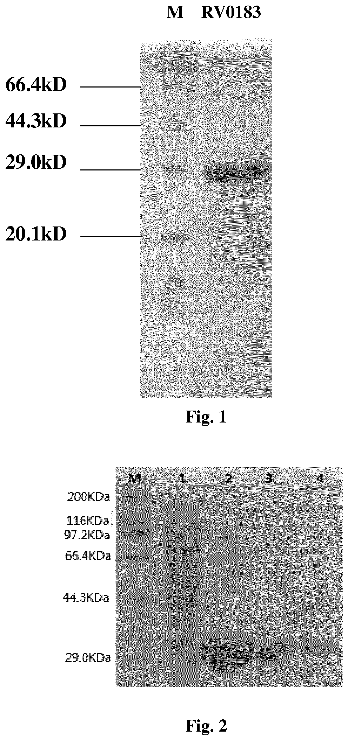

Fig. 1 illustrates the result of SDS-PAGE analysis of the recombinant antigen RV0183 obtained by two-step purification using Ni-NTA and DEAE column after effective expression in *E. coil* ER2566, wherein, Lane 1 represents protein molecular weight standard, and Lane 2 represents the protein RV0183 after two-step purification.
Fig. 2 illustrates the result of SDS-PAGE analysis of the recombinant antigen Plcd obtained by Ni-NTA purification and inclusion body renaturation after effective expression in *E. coil* ER2566, wherein, Lane M represents protein molecular weight standard, Lane 1 represents denatured inclusion bodies, Lane 2 represents the protein Plcd after affinity column chromatography, and Lane 4 represents the protein Plcd after dialysis and renaturation.

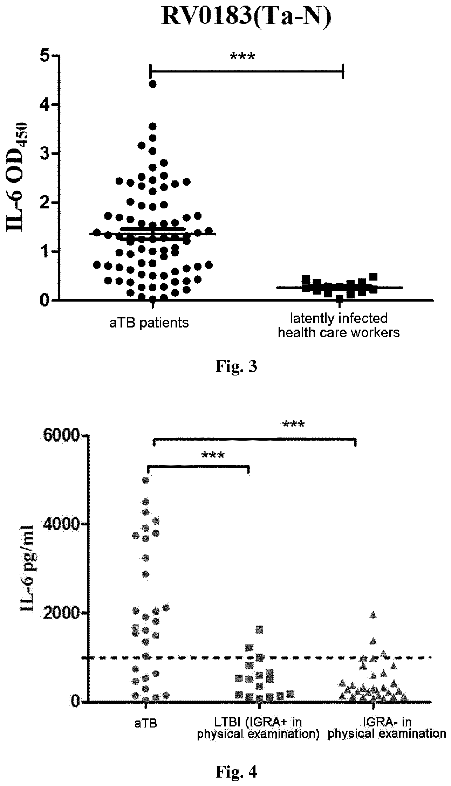

Fig. 3 shows the analysis result of the IL-6 level in whole blood of subjects stimulated with recombinant antigen RV0183. After stimulation of 500 µl whole blood with 1 µg recombinant antigen RV0183 for 20±2 h, plasma samples were 2-fold diluted. The result shows that the IL-6 secretion level in aTB patients was significantly higher than that in a latently infected population. (Note: *** represents p<0.001)
Fig. 4 shows the analysis result of the IL-6 level in whole blood of subjects stimulated with recombinant antigen RV0183. After stimulation of 1 ml whole blood with 2 µg recombinant antigen RV0183 for 20±2 h, plasma samples were 5-fold diluted. The result shows that the IL-6 secretion level in aTB patients was significantly higher than that in a population latently infected with *M. tuberculosis* (LTBI (IGRA+ in physical examination)) and that in a non-infection population (IGRA- in physical examination). (Note: *** represents p<0.001)

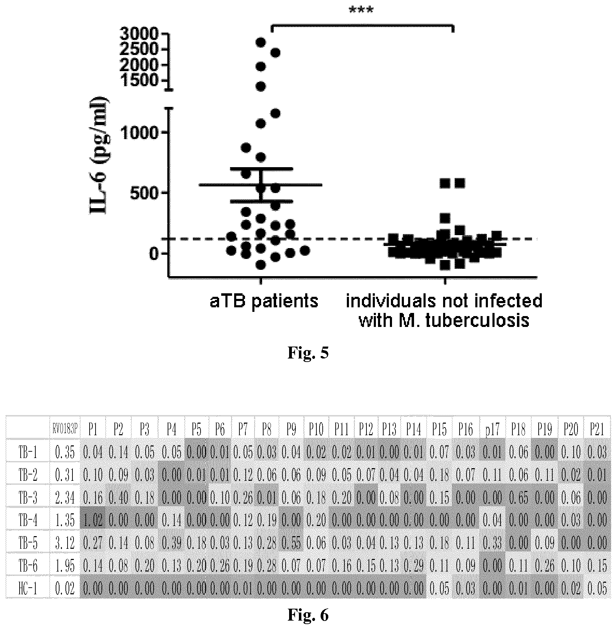

Fig. 5 shows the analysis result of the IL-6 level in whole blood of subjects stimulated with recombinant antigen RV0183. After stimulation of 1 ml whole blood with 2 µg recombinant antigen RV0183 for 20±2 h, plasma samples were 5-fold diluted. The result shows that the IL-6 secretion level in aTB patients in clinic was significantly higher than that in individuals not infected with *M. tuberculosis.* (Note: *** represents p<0.001)
Fig. 6 shows the analysis result of the IL-6 level in whole blood of subjects stimulated with the antigenic fragments of RV0183. The result shows that all of the 21 antigenic fragments of RV0183 could enhance the IL-6 secretion level in whole blood of aTB patients by *in vitro* stimulation, while in healthy control, there was almost no IL-6 response, and green-to-red indicates an increase in intensity of response.

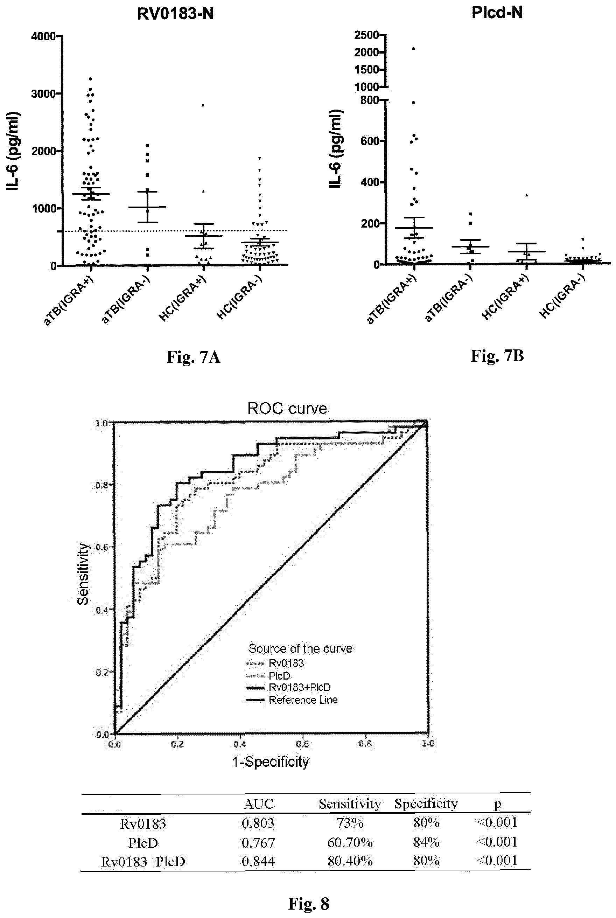

Figs. 7A-7B show the analysis results of the IL-6 level in whole blood of subjects stimulated with recombinant antigen RV0183 and recombinant antigen PlcD, respectively. After stimulation of 1 ml whole blood with 2 µg recombinant antigen RV0183 or 2 µg recombinant antigen Plcd for 20±2 h, plasma samples were 5-fold diluted. The results show that after stimulation with recombinant antigen RV0183 or recombinant antigen PlcD, the IL-6 secretion level in aTB patients was significantly higher than that in a latently infected population and a non-infection population, wherein aTB represents active tuberculosis, HC represents healthy control.
Fig. 8 shows the ROC analysis result of the assay using recombinant antigen RV0183 and recombinant antigen Plcd in combination. The result shows that the assay using recombinant antigen RV0183 and recombinant antigen Plcd in combination could enhance the sensitivity and specificity for detecting aTB patients.

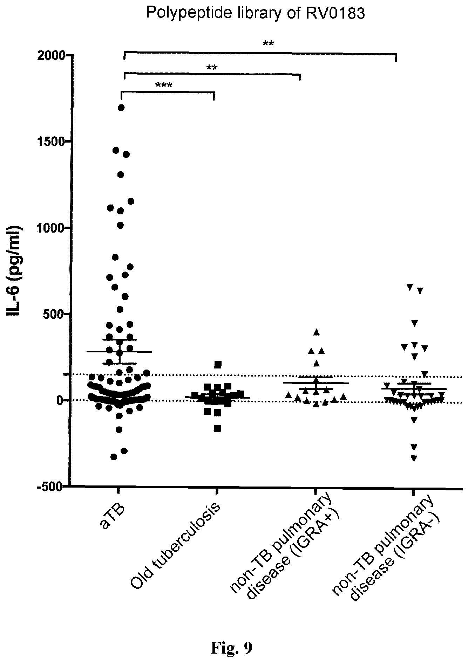

Fig. 9 shows the analysis result of the IL-6 level in whole blood of subjects stimulated with a polypeptide library of RV0183. Whole blood samples from TB patients in clinic, individuals with old tuberculosis, and patients with a non-TB pulmonary disease were co-stimulated with a polypeptide library consisting of 21 antigenic fragments of RV0183. The result shows that the IL-6 secretion level in aTB patients was significantly higher than that in non-aTB samples. (Note: ** represents p<0.01, *** represents p<0.001)

### Sequence information

The information of the sequences involved in the invention are provided in the following Table 1.

**Table 1: Sequence information**

| SEQ ID NO: | Description |
|---|---|
| 1 | the amino acid sequence of recombinant protein RV0183 |
| 2 | the nucleotide sequence of recombinant protein RV0183 |
| 3 | the amino acid sequence of recombinant protein Plcd |
| 4 | the nucleotide sequence of recombinant protein Plcd |
| 5∼25 | the peptide sequences (P1-P21) of a polypeptide library of RV0183 |
| 26∼29 | primers |

The amino acid sequence of recombinant protein RV0183 (SEQ ID NO: 1)

MTTTRTERNFAGIGDVRIVYDVWTPDTAPQAVVVLAHGLGEHARRYDHVAQRLG
AAGLVTYALDHRGHGRSGGKRVLVRDISEYTADFDTLVGIATREYPGCKRIVLGHSMG
GGIVFAYGVERPDNYDLMVLSAPAVAAQDLVSPVVAVAAKLLGVVVPGLPVQELDFTA
ISRDPEVVQAYNTDPLVHHGRVPAGIGRALLQVGETMPRRAPALTAPLLVLHGTDDRLI
PIEGSRRLVECVGSADVQLKEYPGLYHEVFNEPERNQVLDDVVAWLTERL

The nucleotide sequence of recombinant protein RV0183 (SEQ ID NO: 2)

GGATCCATGACTACCACCCGGACTGAACGGAATTTCGCGGCATCGGCGATGT
GCGCATCGTCTACGACGTCTGGACGCCGGACACCGCGCCGCAAGCGGTGGTCGTGC
TGGCCCATGGTCTGGGCGAGCATGCCCGCCGCTACGACCATGTCGCGCAGCGGCTCG
GCGCGGCCGGCCTGGTCACCTATGCGCTTGACCACCGCGGGCATGGCCGCTCGGGTG
GCAAACGGGTGCTAGTGAGAGACATCTCCGAGTACACCGCTGACTTCGACACCCTC
GTTGGGATCGCCACCCGGGAATATCCCGGGTGCAAGCGCATCGTGCTCGGGCACAG
CATGGGCGGCGGCATTGTGTTCGCTTACGGTGTCGAACGTCCAGACAACTACGACCT
GATGGTGCTTTCGGCGCCGGCGGTGGCGGCACAGGACCTGGTGAGCCCGGTAGTGG
CGGTTGCCGCCAAGCTTCTGGGCGTCGTGGTGCCCGGCCTGCCGGTGCAGGAACTGG
ATTTTACTGCCATCTCTCGCGACCCTGAGGTGGTCCAGGCTTACAACACCGACCCAC
TCGTGCACCACGGACGGGTTCCGGCCGGGATTGGCCGCGCGCTGCTGCAGGTGGGC
GAGACCATGCCGCGGCGAGCACCGGCATTGACCGCGCCGCTGCTAGTGCTGCACGG
CACCGATGACCGGCTGATCCCCATCGAGGGCAGCCGTCGCCTGGTCGAATGTGTGG
GATCGGCCGACGTGCAGCTGAAGGAGTATCCCGGGCTGTACCACGAGGTGTTCAAC
GAGCCGGAGCGCAACCAGGTGCTCGACGATGTGGTCGCCTGGCTCACCGAGCGGTT
GTAAGAATTC

The amino acid sequence of recombinant protein Plcd (SEQ ID NO: 3)

DAGVSWKVYRNKTLGPISSVLTYGSLVTSFKQSADPRSDLVRFGVAPSYPASFAAD
VLANRLPRVSWVIPNVLESEHPAVPAAAGAFAIVNILRILLANPAVWEKTALIVSYDENG
GFFDHVVPATAPAGTPGEYVTVPDIDQVPGSGGIRGPIGLGFRVPCFVISPYSRGPQMVH
DTFDHTSQLRLLETRFGVPVPNLTAWRRSVTGDMTSTFNFAVPPNSSWPNLDYPGLHAL
STVPQCVPNAALGTINRGIPYRVPDPQIMPTQETTPTRGIPSGPC

The nucleotide sequence of recombinant protein Plcd (SEQ ID NO: 4)

GATGCCGGCGTCAGCTGGAAGGTGTATCGCAACAAGACACTCGGGCCCATCTC
CTCGGTTCTTACTTACGGCTCGCTTGTGACGTCTTTCAAACAGTCAGCCGATCCCAGG
TCAGATCTTGTCCGCTTTGGCGTGGCACCAAGCTATCCCGCGAGCTTCGCGGCCGAC
GTCTTAGCCAATAGACTGCCGCGGTCTCCTGGGTGATTCCCAATGTTCTCGAATCC
GAACATCCTGCGGTTCCAGCCGCGGCCGGGGCTTTCGCAATCGTCAACATCTTAAGA
ATATTGCTTGCCAATCCTGCGGTGTGGAAAAGACGGCGCTGATCGTCAGCTACGAC
GAAAACGGCGGCTTTTTCGACCACGTTGTTCCTGCTACCGCGCCGGCCGGGACTCCC
GGCGAATATGTCACGGTGCCTGACATCGATCAGGTGCCGGGCTCCGGCGGAATACG
CGGGCCGATCGGTTTGGGCTTTCGCGTTCCCTGCTTCGTCATTTCGCCGTACAGCCGT
GGCCCGCAGATGGTTCACGACACGTTTGACCACACCTCACAGCTGAGATTGCTCGAA
ACTCGGTTCGGGGTGCCAGTTCCCAACCTCACGGCTTGGCGGCGCAGTGTGACCGGC
GACATGACGTCAACGTTCAACTTCGCTGTCCCGCCCAACTCATCATGGCCCAACCTG
GATTATCCCGGGCTGCACGCGCTATCAACGGTGCCGCAGTGCGTGCCCAACGCGGC
GCTGGGCACGATAAACCGTGGAATCCCGTATCGGGTTCCTGATCCACAGATCATGCC
CACGCAGGAAACCACGCCTACCCGTGGTATTCCGAGCGGTCCGTGCTAA

The peptide sequences (P1-P21) (SEQ ID NO: 5∼25) of a polypeptide library of RV0183
RV0183-p1: MTTTRTERNFAGIGDVRIVY (SEQ ID NO: 5)
RV0183-p2: GDVRIVYDVWTPDTAPQAVV (SEQ ID NO: 6)
RV0183-p3: TAPQAVVVLAHGLGEHARRY (SEQ ID NO: 7)
RV0183-p4: GEHARRYDHVAQRLGAAGLV (SEQ ID NO: 8)
RV0183-p5: LGAAGLVTYALDHRGHGRSG (SEQ ID NO: 9)
RV0183-p6: RGHGRSGGKRVLVRDISEYT (SEQ ID NO: 10)
RV0183-p7: RDISEYTADFDTLVGIATRE (SEQ ID NO: 11)
RV0183-p8: VGIATREYPGCKRIVLGHSM (SEQ ID NO: 12)
RV0183-p9: IVLGHSMGGGIVFAYGVERP (SEQ ID NO: 13)
RV0183-p10: AYGVERPDNYDLMVLSAPAV (SEQ ID NO: 14)
RV0183-p11: VLSAPAVAAQDLVSPVVAVA (SEQ ID NO: 15)
RV0183-p12: SPVVAVAAKLLGVVVPGLPV (SEQ ID NO: 16)
RV0183-p13: VVPGLPVQELDFTAISRDPE (SEQ ID NO: 17)
RV0183-p14: AISRDPEVVQAYNTDPLVHH (SEQ ID NO: 18)
RV0183-p15: TDPLVHHGRVPAGIGRALLQ (SEQ ID NO: 19)
RV0183-p16: IGRALLQVGETMPRRAPALT (SEQ ID NO: 20)
RV0183-p17: RRAPALTAPLLVLHGTDDRL (SEQ ID NO: 21)
RV0183-p18: HGTDDRLIPIEGSRRLVECV (SEQ ID NO: 22)
RV0183-p19: RRLVECVGSADVQLKEYPGL (SEQ ID NO: 23)
RV0183-p20: LKEYPGLYHEVFNEPERNQV (SEQ ID NO: 24)
RV0183-p21: EPERNQVLDDVVAWLTERL (SEQ ID NO: 25)
Primers (5'-3') (SEQ ID NOs: 26∼29)
*plcD-1-F:* TTCAACCATCGCCGCCTCTACCA (SEQ ID NO: 26)
*plcD-*1-R: CCATCGCCGCCTCTACCAGT (SEQ ID NO: 27)
*plcD-*2-F: GGATCCATGGATGCCGGCGTCAG (SEQ ID NO: 28)
*plcD-2-R:* AAGCTTTTAGCACGGACCGCTCG (SEQ ID NO: 29)

### Specific modes for carrying out the invention

The present invention is illustrated by reference to the following examples (which are not intended to limit the protection scope of the present invention).

Unless indicated otherwise, the experiments and methods (for example, molecular biological experimental methods and immunological assays) as described in Examples are substantively carried out by conventional methods as well known in the art and as described in various reference documents. See, for example, Sambrook et al., Molecular Cloning: A Laboratory Manual, 2nd edition, Cold Spring Harbor Laboratory Press, Cold Spring Harbor, N.Y. (1989); and Ausubel et al., Current Protocols in Molecular Biology, Greene Publishing Associates (1992), which are incorporated herein by reference. Enzymatic reactions and purification technologies are carried out in accordance with the instructions of manufacturers, for example, as generally used in the art or described herein. Reagents or instruments, the manufacturers of which are not indicated, are the conventional products that can be purchased on the market. Those skilled in the art understand that the examples are used for illustrating the present invention, but not intended to limit the protection scope of the invention. All the disclosed solutions and other reference documents as mentioned here are incorporated herein by reference.

### Example 1. Cloning, expression and endotoxin-removal purification of recombinant protein RV0183

### 1. Construction of an expression vector comprising a sequence encoding recombinant protein RV0183

### 1.1.1 Obtainment of a gene fragment of interest

A gene fragment of interest was artificially synthesized. In order to introduce an expression vector conveniently, when the gene fragment of interest was synthesized, an enzyme cleavage site for BamHI was added at 5' end, and an enzyme cleavage site for EcoRI was added at 3' end. The vector used was pMD18T. Therefore, a RV0183 plasmid containing enzyme cleavage sites at two ends was obtained.

Enzyme cleavage: the artificially constructed RV0183 plasmid was subjected to double enzyme digestion by BamHI/EcoRI. The enzyme cleavage product was recovered by agarose gel electrophoresis.

### 1.1.2 Fusion of a gene fragment of interest and construction of an expression vector

The pTO-T7 vector (the information of which can be found in Luo Wenxin, Zhang Jun, Yang Haijie, et al., Construction and application of an Escherichia coli high effective expression vector with an enhancer, Chinese Journal of Biotechnology, 2000, 16(5): 578-581), which had been subjected to double enzyme digestion by BamHI/EcoRI, was ligated to the enzyme cleavage product encoding RV0183, thereby obtaining an expression vector pTO-T7-RV0183 comprising the gene fragment of RV0183.

### 2. Expression and endotoxin-removal purification of recombinant protein RV0183

*E. coli* ER2566 (preserved in laboratory) transformed with the constructed expression vector pTO-T7-RV0183, were spread on a solid LB medium (the components of the LB medium: 10 g/L peptone, 5 g/L yeast powder, and 10 g/L NaCl, the same hereinafter) containing kanamycin (Kan, at a final concentration of 100 µg/ml). Stationary culture was carried out at 37 °C until single colonies were observed clearly. Single colonies were picked and transferred to a liquid LB medium (containing 100 µg/ml kanamycin). The cultures were incubated in a shaking incubator at 180 rpm at 37 °C for 8 h. Then the bacteria solution was transferred to 500 mL of liquid LB medium (containing 100 µg/ml kanamycin) in a culture flask, and incubated in a shaking incubator at 180 rpm at 37 °C. When the OD₆₀₀ of the liquid in the culture flask reached about 0.6-0.8, IPTG was added at a final concentration of 0.2 mM/L, and the incubation was further performed at 180 rpm for 4 h at 37 °C. After centrifugation of the cultures at 5000 rpm for 10 min, the bacteria were collected.

Since recombinant protein RV0183 was used to stimulate whole blood, endotoxin needed to be removed from the protein product. Methods for purifying a recombinant protein and removing endotoxin from bacterial proteins are well known by a person skilled in the art. In the Example, the following exemplary method was used.

The collected bacteria were suspended in a buffer (50 mM TB8.0), placed in an ice bath, disrupted by ultrasonic waves, and then centrifuged at 12000 rpm for 10 min, and inclusion bodies were collected.

Ni-NTA column purification: the supernatant of the expressed protein of interest was purified by self-assembled Ni-NTA column (manufacturer of media: Qiagen). In brief, the sample was loaded to a Ni-NTA column, and eluted with 0.2% sodium deoxycholate, 50 mM TB8.0 to remove a part of endotoxin, followed by elution with 200 ml 50 mM TB8.0 to remove sodium deoxycholate; finally, the protein of interest was eluted with 100 ml eluent (150 mM imidazole, 50 mM TB8.0).

DEAE column purification: the eluted protein was further purified by DEAE column. The loading buffer was 50 mM TB8.0, and the eluent was 400 mM NaCl, 50 mM TB8.0. The eluted protein was dialyzed to 50 mM TB8.0 and stored.

During purification of the protein, the buffers used were prepared with water for injection, and the experimental containers used were dried and baked at 200 °C for more than 2h.

The recombinant protein RV0183 obtained by two-step purification was detected by SDS-PAGE, and the result was shown in Fig. 1. The result showed that recombinant protein RV0183 had a molecular weight of about 30 kD, and after the two-step purification, the recombinant protein RV0183 had a purity of above 95%.

A limulus reagent was used to detect endotoxin in the purified recombinant protein RV0183. The result showed that after the two-step purification, the recombinant protein RV0183 had an endotoxin content of below 100 EU/mg.

### Example 2. Cloning, expression and endotoxin-removal purification of recombinant protein Plcd

### 2.1 Construction of an expression vector comprising a sequence encoding recombinant protein PlcD

The gene fragment of interest was obtained by PCR amplification.

Since the genomic fragment of *M. tuberculosis* was long and the GC-base content was higher, it was difficult to amplify a fragment of interest directly. Therefore, a fragment of interest was obtained by a Nested PCR method through two rounds of PCR amplification using the genomic DNA of *M. tuberculosis* H37Rv as template.

### 2.1.1 Design of PCR primers

According to the gene sequence corresponding to the PlcD protein of *M. tuberculosis* H37Rv as recorded in NCBI, primers were evaluated and screened by software, and the corresponding enzyme cleavage sites were added. The designed primers were shown as followed:

**Table 2: Primer sequence (SEQ ID NOs: 26-29**

| SEQ ID NO: | Primer name | Primer sequence | Enzyme cleavage site |
|---|---|---|---|
| 26 | *plcD-1-F* | 5'-TTCAACCATCGCCGCCTCTACCA-3' | no |
| 27 | *plcD-1-R* | 5'-CCATCGCCGCCTCTACCAGT-3' | no |
| 28 | *plcD-2-F* | 5'-GGATCCATGGATGCCGGCGTCAG-3' | BamH I |
| 29 | *plcD-2-R* | 5'-AAGCTTTTAGCACGGACCGCTCG-3' | Hind III |

### 2.1.2 First round of PCR amplification reaction

In accordance with the following table, the components were homogeneously mixed in a 0.5 ml Ependorf tube, and a 20 µl PCR reaction system was established.

### First-Round PCR reaction system for plcD protein-coding gene

| *plcD-*1-F | *plcD-1-R* | 10xBuffer | dNTP | DMSO | glycerol | rTaq | template DNA | ddH₂O |
|---|---|---|---|---|---|---|---|---|
| 0.4 µL | 0.4 µL | 2 µL | 0.8 µL | 1.5 µl | 1.5 µl | 0.8 µL | 0.4 µL | 12.2 µL |

Amplification program: pre-denaturation at 95 °C for 10 min; after hot start, 30 cycles of denaturation at 95 °C for 1min, annealing at 55 °C for 1min, and elongation at 72 °C for 3 min; elongation at 72 °C for 10 min.

### 2.1.3 Second round of PCR amplification reaction

The First-Round PCR product was used as template, and in accordance with the following table, the components were homogeneously mixed in a 0.5 ml Ependorf tube to form a 50 µl PCR reaction system.

### Second-Round PCR reaction system for plcD protein-coding gene

| *plcD-*2-F | *plcD-2-R* | 10xBuffer | dNTP | DMSO | glycerol | rTaq | template DNA | ddH₂O |
|---|---|---|---|---|---|---|---|---|
| 1 µl | 1 µl | 5 µl | 2 µL | 2.5 µl | 2.5 µl | 2 µL | 1 µl | 33 µL |

Amplification program: pre-denaturation at 95 °C for 10 min; 30 cycles of denaturation at 95 °C for 1 min, annealing at 55 °C for 1 min, and elongation at 72 °C for 3 min; elongation at 72 °C for 10 min. The amplification product was identified and recovered by 1% agarose gel electrophoresis.

### 2.1.4 Construction of a cloning vector

The following components were added and mixed in a 0.5 ml Ependorf tube, and reacted at 16 °C for 12 h, thereby obtaining a pMD18-T cloning vector ligated to the gene fragment of interest.

| pMD18-T Vector | Gene fragment of interest | Solution I | Total reaction volume |
|---|---|---|---|
| 0.5 µl | 5 µl | 4.5 µl | 10 µl |

### 2.1.5 Transformation with the cloning vector-ligated product

40 µl competent DH5α cell and 10 µL of the cloning vector-ligated product were mixed, and incubated on ice for 15 min to allow adsorption. After heat shock at 42 °C for 90 s, the reaction was immediately stopped by virtue of an ice bath. 200 µL antibiotic-free LB medium was added, and the cultures were incubated in a shaking incubator at 37 °C for 45 min. 200 µL culture solution was spread onto a solid medium containing ampicillin, and the cultures were incubated at 37 °C for 12 h.

### 2.1.6 Recovery of the cloning vector plasmid

Single clone colonies were picked and seeded in 5 mL liquid LB medium containing ampicillin, and incubated at 37 °C in a shaking incubator overnight. 1.5 mL bacterial solution was placed in a 1.5 mL Eppendorf tube, and centrifuged at 12000g for 1 min, and the supernatant was discarded. 250 µL Solution I was added, and the precipitate was completely suspended under shaking. 250 µL Solution II was added, and the tube was gently turned upside down for several times so that bacteria were lysed. 350 µL Solution III was added, and the resultant mixture was mixed homogeneously, and placed in an ice bath for 15 min. After centrifugation at 12000 rpm for 10 min, the supernatant was transferred to an equilibrated adsorption column for plasma-recovery, and was left to stand at room temperature for 5 min. The subsequent recovery operations were the same as the steps in gel extraction.

The recovered product was identified by 1% agarose gel electrophoresis, and it was confirmed that the recovery was successful. The obtained pMD18-T containing the gene fragment of interest was cleaved by enzyme, to recover the cloned fragment of interest, and a part of cloning vectors was sent to be sequenced.

### 2.1.7 Construction of the cloning vector expressing PlcD

The gene fragment of interest obtained by enzyme cleavage of pMD-18T was ligated to the enzymatically cleaved PTO-T7 vector, and the ligation system was as followed: PTO-T7 vector (1 µl), the gene fragment of interest (6 µl), 10xT4 DNA Ligase Buffer (1 µl), and T4 DNA ligase (1 µl), at a total reaction volume of 10 µl. The time for ligation was 2 h.

### 2.2 Transformation with the cloning vector for expression and protein expression and purification

### 2.2.1 Transformation with the expression vector-ligated product

The steps were the same as the above-mentioned ligation steps, ER2566 competent cells were used for transformation, and the transformed ER2566 was spread onto a kanamycin-containing LB resistant medium.

### 2.2.2 Expression and purification of Plcd protein

### Extraction of the protein of interest

The bacteria, which were identified to express the correct protein of interest in a small amount, were activated. After incubation at 37 °C for 4 h, 1 mmol/L IPTG was added, and the culture was maintained at 37 °C for 4 h. The bacteria were collected by centrifugation at 9000g for 6 min. The bacteria were rinsed and then re-centrifuged to collect the precipitate. The precipitate was re-suspended at a ratio of 200 mL bacterial solution to 5 mL TB 8.0 buffer. The re-suspended bacterial solution was disrupted by ultrasonic treatment, and the time for treatment was calculated at 3 min per 500 mL bacterial solution. The sample after ultrasonic treatment was centrifuged at 12000g for 10 min to collect the disrupted precipitate. The disrupted product precipitate was rinsed with TB 8.0 solution repeatedly for three times, and was subjected to shaking in a 37 °C incubator for 15 min after each suspension. After re-suspending in 15 mL TB 8.0 solution containing 6 M urea, the precipitate was blew up sufficiently. When most of the precipitate was blew up, centrifugation at 12000g for 10 min was performed to collect the supernatant, which was used in the following purification step.

### Nickel ion column affinity chromatography

Ni-NTA column purification: the supernatant for expression of the protein of interest was purified by a self-assembled Ni-NTA column (manufacturer of media: Qiagen). The sample was loaded onto a Ni-NTA column, eluted with 0.2% sodium deoxycholate, 50 mM TB8.0+6 M urea to remove a part of endotoxin, and then washed with 200 ml 50 mM TB8.0+6 M urea to remove sodium deoxycholate; and finally, the protein of interest was eluted with 100 ml of the eluent (150 mM imidazole, 50 mM TB8.0, 6 M urea).

### Renaturation of protein

plcD protein was expressed in the form of inclusion bodies. Therefore, in the experiment, 6 M urea was used to denaturalize and dissolve the inclusion bodies, and after affinity-chromatographic purification, the denaturant was gradually removed by using a gradient dialysis method. During the dialysis, dithiothreitol (DTT) was added to prevent the formation of wrong disulfide bonds, help the correct folding of protein, and retain the soluble state. The dialysis steps were shown in the following table. The result was shown in Fig. 2.

### Steps for dialysis and renaturation of plcD protein

| Dialysis Solution | Components | Dialysis time |
|---|---|---|
| Dialysis Solution I | 4 M urea in TB 8.0, 2 mmol/L DTT | 4 h |
| Dialysis Solution II | 2 M urea in TB8.0, 2 mmol/L DTT | 12 h |
| Dialysis Solution III | 1 M urea in TB8.0, 2 mmol/L DTT | 4 h |
| Dialysis Solution IV | TB 8.0, 1 mmol/L DTT | 4 h |
| Dialysis Solution V | TB 8.0 | 4 h |

### Removal and detection of endotoxin

According to the method of Shigui Liu *et al.* (Liu SG, Tobias R. Removal of endotoxin from recombinant protein preparations [J]. Clinical Biochem, 1997, 30: 455-463), endotoxin was removed from the recombinant protein by extraction using a non-ionic detergent in the experiment

Triton X-114 was added into the protein solution at a final concentrating of 1% (w/v), and the two phases were mixed homogeneously at 4 °C. After shaking for 15 min, the protein solution containing Triton X-114 was placed in a 37 °C water bath for 10 min, a turbid system was observed, and oil droplets appeared in the solution. After centrifugation at 25 °C 12000g for 10 min, water phase was removed carefully. The above steps were repeated once to collect water phase. The endotoxin content in the treated protein solution was determined by limulus reagent gel method.

### Example 3. Screening of markers for RV0183-specific T cell response

### 3.1 Collection and treatment of stimulated samples

### 3.1.1 Collection of samples for in vitro stimulation

Heparin-anticoagulation tubes were used to collect 5 ml peripheral blood from 4 hospitalized aTB patients who were tested positive in IGRAs, and collect 5 ml peripheral blood from 4 latently infected individuals who were tested positive in IGRAs and had no clinical symptoms.

### 3.1.2 Stimulation and Collection of samples

The samples were dispensed into 9 endotoxin-free 2 ml EP tubes, at 500 µl peripheral blood per EP tube, and to each of the EP tubes, a culture solution (15 µl) was added (the culture solution was prepared as followed: to 30 µl PBS, 133.33 mg/ml D-glucose and 166.67 mM KCl were added; wherein PBS was prepared as followed: to Na₂HPO4·12 H₂O 2.9 g , KH₂PO4 0.24 g, NaCl 8 g, and KCl 0.2 g, ultrapure water was added to a final volume of 1 L.). The EP tubes were designated as N, Ta, and P, wherein the N tube did not contain any stimulating agent, the Ta tube contained the stimulating antigen RV0183 (1 µg), and the P tube contained PHA (20 µg).

After adding peripheral blood to the EP tube, the resultant mixture was mixed homogenously by turning the tube upside down, and was incubated in a 37 °C incubator for 20-24 h; plasma samples were collected at 5000 rpm, and the cytokine contents in plasma were determined.

### 3.2 Determination of cytokine contents in plasma

The cytokines in the collected plasma samples were quantitated by using the commercialized Milliplex Kit (Merck Millipore, St.Charles, Missouri, USA) (Batch No: HCYTMAG-60K-PX38, HCP2MAG-62K-PX23, HCP3MAG-63K-PX11), wherein the detection indexes comprised 69 cytokines including EGF (see Table 2), and the test platform was Luminex 200 (the washing solution, standard sample, reference sample, and magnetic beads in the following steps were obtained from the Kit).

The test steps comprised: 1) adding a washing solution (200 µl) to each well in a 96-well test plate, shaking at room temperature for 10 min, and removing the washing solution; 2) to the corresponding wells, separately adding a standard sample (25 µl) and a reference sample (25 µl), wherein two wells were set for each sample; 3) adding an assay buffer (25 µl) to each sample well; 4) to each of the standard sample wells, the reference sample wells and the blank control sample wells, adding a plasma matrix (25 µl) (the matrix refers to a sample diluent in the Kit); 5) adding a plasma sample (25 µl) to each well; 6) adding magnetic beads (25 µl) to each well, and incubating at 2-8 °C overnight; 7) removing the liquid in the well, washing the well twice at 200 µl washing solution per well; 8) adding a detection antibody (25 µl) to each well, and incubating at room temperature for 1 h; 9) adding Steptavidin-Phycoerythrin (25 µl) to each well, and reacting at room temperature for 30 min; 10) removing the liquid in the well, and washing the plate twice; 11) adding a sheath fluid (150 µl) to each well, and mixing homogeneously under shaking; 11) reading the plate in Luminex 200 platform; and 12) for each sample, subtracting the actually determined value of the N tube from the actually determined value of the Ta tube to obtain a difference value as a final result (i.e. the actually increased value of cytokine, represented by Ta-N). (The method was a double antibody sandwich method)

The result was shown in Table 3. The cytokines were analyzed for their different responses in aTB patients and a latently infected population, and it was found by the screening that IL-6 could effectively determine whether individuals with TB infection were in a state of active tuberculosis (aTB) or in a state of Latent Tuberculosis Infection (LTBI) (the hospitalized TB patients with clinical symptoms were aTB patients, and the subjects, which were tested positive in IGRA and had no clinical symptoms, were the latently infected individuals).

**Table 3**

| | | Sample 1(aTB) | Sample 2(aTB) | Sample 3(aTB) | Sample 4(aTB) | Sample 5 (LTBI) | Sample 6(LTBI) | Sample 7 (LTBI) | Sample 8 (LTBI) |
|---|---|---|---|---|---|---|---|---|---|
| | | Stimulating antigen RV0183 | | | | | | | |
| EGF | pg/ml | -15 | -47 | 15.29 | 16.45 | -19 | -32 | 10 | -5 |
| FGF-2 | pg/ml | 3.42 | 21.6 | 25.42 | 7.51 | -1.93 | 15.95 | -1 | 2.7 |
| EOTAXIN | pg/ml | 13 | -7 | -3 | 7 | -18 | 10 | 10 | -36 |
| TGF-a | pg/ml | 0.97 | 5.42 | 5.71 | 2.9 | 0.1 | 0.97 | 7.5 | 0.09 |
| G-CSF | pg/ml | 146.96 | 282.14 | 125 | 110.96 | 118.4 | 134.96 | -65 | 22.21 |
| Flt-3L | pg/ml | 0 | 0 | 0 | 0 | 0 | 0 | 0 | 0 |
| GM-CSF | pg/ml | 18.82 | 71.82 | 30.3 | 16.75 | 17.17 | 26.81 | 16.41 | 26.15 |
| Fractalkine | pg/ml | 103 | 174 | 152.3 | 183.68 | 160.64 | 8 | 50 | 81.64 |
| IFNa2 | pg/ml | 20.11 | 43.99 | 0 | 14.08 | -3.45 | 17.61 | 13.32 | 17.21 |
| IFN-g | pg/ml | 14.74 | 16.89 | 2.03 | 5.81 | -0.42 | 1.5 | 5.54 | 57.59 |
| GRO | pg/ml | 3362 | 2571 | 2792 | 461 | 344 | 3095 | -4242 | 179 |
| IL-10 | pg/ml | 55.65 | 70.29 | 0 | 9.39 | 4.07 | 0 | 5.4 | 0 |
| MCP-3 | pg/ml | 494.02 | 9772.1 | 763 | 294.42 | 93.3 | 440.39 | 2137 | 192.95 |
| IL-17P40 | pg/ml | 0 | 0 | 0 | 0 | 0 | 0 | 0 | 0 |
| MDC | pg/ml | -100 | 57 | 9.24 | 34.53 | -255 | 115 | -12 | 40 |
| IL-12P70 | pg/ml | 0 | 1.07 | 0.42 | 0 | 0 | 0.21 | 0.11 | 0 |
| IL-13 | pg/ml | 0 | 0 | 0 | 0 | 0 | 0 | 0 | 0 |
| IL-15 | pg/ml | 0 | 0 | 0 | 0 | 0 | 0 | 0 | 0 |
| sCD40L | pg/ml | 885 | -1481 | 1567 | 636 | -636 | -1707 | -2639 | -2161 |
| IL-17A | pg/ml | 0 | 1.35 | 0 | 0 | 0 | 0 | -0.4 | 0 |
| IL-1RA | pg/ml | 0 | 45.6 | 0 | 0 | 0.39 | 0 | 15.05 | 0 |
| IL-1a | pg/ml | 29.62 | 83.48 | 66.36 | 31.88 | 0 | -24.77 | 38.26 | 3.61 |
| IL-9 | pg/ml | 0 | 0 | 0 | 0 | 0 | 0 | 0 | 0 |
| IL-1b | pg/ml | 13.78 | 132.92 | 47.86 | 78.77 | 59.26 | 53.93 | 5.65 | 29.01 |
| IL-2 | pg/ml | 0 | 0 | 0 | 0 | 0 | 0 | -61.6 | 0 |
| IL-3 | pg/ml | 0 | 0 | 0 | 0 | 0 | 0 | 0 | 0 |
| IL-4 | pg/ml | -9.92 | 0 | 0 | 0 | 0 | 0 | 0 | 0 |
| IL-5 | pg/ml | 0 | 0 | 0 | 0 | 0 | 0 | -2.76 | 0 |
| IL-6 | pg/ml | 790 | 6254.3 | 761.01 | 1098.7 | 403.11 | 241.52 | 329 | 248.01 |
| IL-7 | pg/ml | 7.81 | 19.08 | 16.88 | 15.1 | 7.53 | 11.7 | 2.8 | 2.08 |
| IL-8 | pg/ml | 5505.88 | 5694.9 | 5889.9 | 6290.9 | 6002.9 | 6015.9 | 0 | 460 |
| IP-10 | pg/ml | 7761.01 | 0 | 0 | 0 | 589 | -4975 | 0 | 9317 |
| MCP-1 | pg/ml | 6668.62 | 0 | 0 | 6099.6 | 0 | 6729.6 | 0 | 6968.6 |
| MIP-1a | pg/ml | 412 | 3062.9 | 917.75 | 152.9 | 248.53 | 285 | 913 | 138.26 |
| MIP-1b | pg/ml | 515 | 2091 | 422 | 480 | 386 | 394 | 240 | 418 |
| TNF-a | pg/ml | -12.67 | 159.5 | 43 | 56.05 | 63.51 | 39.22 | 49 | 39.33 |
| TNF-b | pg/ml | 0 | 0 | 0 | 0 | 0 | 0 | 0 | 0 |
| VEGF | pg/ml | 153 | 422 | 168 | 107.13 | 19.56 | 104 | 147 | 96.24 |
| CXLC6 | pg/ml | 58 | 267 | 48 | 38.46 | 27 | 240 | 1 | 24.21 |
| CXCL9 | pg/ml | -543 | -1447 | -904 | -10528 | -227 | -779 | -1340 | 1041 |
| CXCL11 | pg/ml | -176 | 87 | -144 | -445.8 | -37 | -150 | -203 | -99 |
| CXCL19 | pg/ml | -21.31 | -9.05 | -28 | -39 | -1.17 | -15.7 | -9.31 | -4.99 |
| CXCL20 | pg/ml | 47.89 | 107.51 | 227.32 | 243.33 | 79.97 | 360.95 | 1.52 | 49.84 |
| IL-11 | pg/ml | 0 | 5.23 | 0.07 | -0.84 | -1.74 | 1.7 | -2.67 | 0 |
| IL-29 | pg/ml | -33 | -217 | -113.5 | -124 | 0 | 0 | 0 | 0 |
| M-CSF | pg/ml | 0 | 0 | 0 | 0 | 0 | 0 | 0 | 0 |
| XCL1 | pg/ml | -24.55 | -9.55 | -19.74 | -33.12 | 4.98 | -46.29 | -19.3 | 0 |
| 6Ckine | pg/ml | 206 | 397 | 424 | 33 | -64 | 94 | 518 | -30.9 |
| BCA-1 | pg/ml | -0.83 | 39.21 | 6.69 | 3.15 | 6.32 | 4.77 | 5.54 | 8.84 |
| CTACK | pg/ml | 167 | -21 | -30 | -29 | -20 | -4 | -18 | -20 |
| ENA-78 | pg/ml | 1853 | 15438 | 2397 | 4530 | 798 | 13933 | 26926 | 256 |
| Eotaxin-2 | pg/ml | -39 | -1498 | 170 | -1087 | -576 | -194 | -272 | -412 |
| Eotaxin-3 | pg/ml | -17.91 | 63 | 142 | 54 | -88 | 8 | -8 | -27 |
| 1-309 | pg/ml | 1.66 | 8.86 | 3.39 | -0.21 | -3.32 | -4.9 | 7.54 | 1.35 |
| IL-16 | pg/ml | -25.14 | -35.82 | 65.44 | -63.86 | -29.7 | -289 | -95.3 | -10.09 |
| IL-20 | pg/ml | 73 | 49 | 917.62 | -113 | -63 | 83 | 446 | 370 |
| IL-21 | pg/ml | 0 | 0 | 0 | 0 | 0 | 0 | 0 | 0 |
| IL-23 | pg/ml | 1.56 | 23.47 | 174 | 0 | 0 | -4.67 | 60.74 | 0 |
| IL-28a | pg/ml | 0 | 18.63 | 9.58 | 2.55 | 0 | -2.55 | 7.7 | -22.32 |
| IL-33 | pg/ml | 0 | 0 | 60.02 | 0 | 2.43 | 0 | 0 | 0 |
| LIF | pg/ml | 0 | 0 | 0 | 13.58 | 0 | 3.76 | 16.1 | 0 |
| MCP-2 | pg/ml | 103.43 | 813 | 5.5 | 70.97 | 36.72 | 12.29 | -19.4 | 133.34 |
| MCP-4 | pg/ml | 10.17 | 43 | 19 | 9.38 | -7.69 | -10.81 | 65 | 9.96 |
| MIP-1d | pg/ml | 191 | 4245 | 119 | -375 | -89 | -1152 | -9 | -564 |
| SCF | pg/ml | 0 | 8.66 | 0.82 | 0 | -5.14 | 0 | 5.77 | 0 |
| SDF-1a | pg/ml | 298 | 1086 | 1564 | -511 | -519 | 0 | 507 | -410 |
| TARC | pg/ml | -0.4 | 1.33 | 0 | 4.88 | -2.55 | -4.64 | -10.1 | -1.11 |
| TPO | pg/ml | 47 | 102 | 169 | -47 | -61 | -71 | 30 | -7 |
| TRAIL | pg/ml | 0 | 31.1 | 0 | 0 | 0 | 0.43 | 3.55 | 0 |

### Example 4. Screening of aTB patients from a TB infection population by using RV0183

### 4.1 Cohort subject information

4.1.1 aTB patients: 79 patients clinically diagnosed with active tuberculosis; 2) a latent tuberculosis infection (LTBI) population and a healthy population: 37 health care workers participated into the cohort, wherein 14 heath care workers, who were tested positive in IGRAs, but had no clinical symptoms of tuberculosis and no history of tuberculosis, were diagnosed as latently infected individuals; and the other 23 heath care workers, who were tested negative in IGRAs, were diagnosed as healthy individuals not infected with *M. tuberculosis.*

### 4.2 Sample collection and a system for sample stimulation and culture

4.2.1 Whole blood samples were dispensed into three 2 ml EP tubes, at 500 µl whole blood per EP tube; the 3 EP tubes were designated as N, Ta and P; the N tube comprised a culture solution (15 µl) (which was prepared as described in Example 4) containing no antigen; the Ta tube comprised a culture solution (15 µl) containing the recombinant antigen RV0183 (1 µg); and the P tube comprised a culture solution (15 µl) containing PHA (20 µg).
4.2.2 After dispensing, the whole blood was mixed homogeneously by turning the tube upside down gently, and was incubated in a 37 °C incubator for 20±2 h.
4.2.3 After incubation, plasma was collected by centrifugation at 5000 rpm for 10 min, and the IL-6 level was determined.

### 4.3 Determination of IL-6 level in plasma

IL-6 ELISA Kit (Xiamen Innovax Biotech CO., LTD.) was used to determine the IL-6 level in plasma.
1) Treatment of plasma samples: the sample was 2-fold diluted with 20% newborn bovine serum (NBS); 2) Sample addition and incubation: to each well of a 96-well IL-6 test plate, the treated plasma (100 µl) was added, the reaction was carried out at 37 °C for 40 min, the plate was washed for five times, and then remove the wash; 3) Enzyme addition and incubation: an enzyme-labeled secondary antibody (100 µl) was added to each well, the reaction was carried out in a 37 °C incubator for 40 min, the plate was washed for five times, and then remove the wash; 4) Color development: a color developing solution was added, the reaction was carried for 10 min, and a stop solution (50 µl) was added to stop the reaction; 5) Determination: the absorption value at 450 nm was determined by an ELISA instrument; and 6) for each sample, subtracting the actually determined value of the N tube from the actually determined value of the Ta tube to obtain a difference value as a final result (i.e. the actually increased value of cytokine, represented by Ta-N).

The result was shown in Fig. 3. 500 µl peripheral blood cells was stimulated with 1 µg recombinant antigen, and there was a significant difference in IL-6 secretion level between aTB patients and a population latently infected with *M. tuberculosis.*

### Example 5. Screening of aTB patients from a general population by using RV0183

### 5.1 Cohort subject information

5.1.1 aTB patients: 207 patients who were clinically diagnosed with active tuberculosis;
5.1.2 Subjects undergoing a health check-up and health care workers: 65 subjects who underwent a health check-up in general department, wherein the subjects, who were tested positive in IGRAs and had no clinical symptoms, were diagnosed as the individuals latently infected with *M. tuberculosis*; and the subjects, who were tested negative in IGRAs, were diagnosed as the individuals not infected with *M. tuberculosis.* Among 82 health care workers, the workers, who were tested positive in IGRAs and had no clinical symptoms, were diagnosed as the individuals latently infected with *M. tuberculosis*; and the workers, who were tested negative in IGRAs, were diagnosed as the individuals not infected with *M. tuberculosis.*

### 5.2 Sample collection and the system for sample stimulation and culture

5.2.1 3 ml peripheral blood was collected from each subject, and the whole blood was dispensed to three 2 ml EP tubes, at 1 ml whole blood per tube; the three EP tubes were designated as N, Ta and P,. To each EP tube, a culture solution (30 µl) (which was prepared as described in Example 4) was added; wherein to the N tube, a culture solution containing no antigen was added; to the Ta tube, a culture solution (30 µl) containing the recombinant antigen RV0183 (2 µg) was added; and, to the P tube, a culture solution (30 µl) containing PHA (40 µg) was added;
5.2.2 After dispensing, the whole blood was mixed homogenously by turning the tube upside down, and incubated statically in a 37 °C incubator for 20±2 h;
5.2.3 After incubation, the EP tubes were placed in a centrifugal machine, plasma samples were collected by centrifugation at 5000 rpm for 10 min, and the IL-6 secretion level was determined;

### 5.3 Determination of IL-6 secretion level in plasma

IL-6 ELISA Kit (Xiamen Innovax Biotech CO., LTD.) was used to determine the IL-6 level in plasma.

The method for determining IL-6 was the same as the one described in Example 4.

The result was shown in Fig. 4. After stimulation of peripheral blood cells with RV0183, there was a significant difference in the IL-6 secretion level between aTB patients and a population latently infected with *M. tuberculosis.* There was also a significant difference in the IL-6 level between aTB patients and individuals not infected with *M. tuberculosis,* and the result was shown in Fig. 5. The above results showed that RV0183 could be used for detecting aTB patients.

### Example 6. Screening of aTB patients by using antigenic fragments of RV0183

### 6.1 Cohort subject information

The same as Example 5.

### 6.2 Sample collection and the system for sample stimulation and culture

6.2.1 aTB subjects in clinic were collected, 15 ml whole blood was collected from each subject, and the whole blood was dispensed to 22 EP tubes, at 500 µl whole blood per tube. Among them, one EP tube was used as a negative control tube (N), and the other 21 EP tubes were designated as PI, P2, P3 ... P21. To the P1 tube, the peptide fragment RV0183-p1 (1 µg) was added; to the P2 tube, the peptide fragment RV0183-p2 (1 µg) was added, and so on, until the peptide fragment RV0183-p21 (1 µg) was added to the P21 tube.
6.2.2 After dispensing, the whole blood was mixed homogenously by turning the tube upside down, and incubated statically in a 37 °C incubator for 20±2 h;
6.2.3 After incubation, the EP tubes were placed in a centrifugal machine, plasma samples were collected by centrifugation at 5000 rpm for 10 min, and the IL-6 secretion level was determined;

### 6.3 Determination of IL-6 secretion level in plasma

IL-6 ELISA Kit (Xiamen Innovax Biotech CO., LTD.) was used to determine the IL-6 level in plasma.

The method for determining IL-6 was the same as the one described in Example 4.

The result was shown in Fig. 6. In the whole blood of aTB patients stimulated with the antigenic fragments (p1-p21) of RV0183, the IL-6 secretion level was increased to different extents, while in healthy control, there was almost no IL-6 response. The result showed that the antigenic fragments could be used for diagnosing active tuberculosis.

### Example 7. Screening of aTB patients by using RV0183 and PIcD in combination

### 7.1 Cohort subject information

aTB(IGRA+) represented patients who were tested positive in TB-IGRA and were diagnosed with active tuberculosis, and aTB(IGRA-) represented patients who were tested negative in TB-IGRA and were diagnosed with active tuberculosis. HC(IGRA+) represented healthy subjects who were tested positive in TB-IGRA, and HC(IGRA-) represented healthy subjects who were tested negative in TB-IGRA. There were 55 subjects for aTB(IGRA+), 9 subjects for aTB(IGRA-), 13 subjects for HC(IGRA+), and 52 subjects for HC(IGRA-).

### 7.2 Sample collection and the system for sample stimulation and culture

7.2.1 4 ml peripheral blood was collected from each subject, and whole blood was dispensed to four 2 ml EP tubes, at 1 ml whole blood per EP tube; the 4 EP tubes were designated as N, Ta1, Ta2, and P, a culture solution (30 µl) (which was prepared as described in Example 4) was added to each EP tube; wherein to the N tube, a culture solution containing no antigen was added; to the Ta1 tube, a culture solution (30 µl) containing the recombinant antigen RV0183 (2 µg) was added; to the Ta2 tube, a culture solution (30 µl) containing recombinant antigen Plcd (2 µg) was added; and to the P tube, a culture solution (30 µl) containing PHA (40 µg) was added;
7.2.2 After dispensing, the whole blood was mixed homogenously by turning the tube upside down, and incubated statically in a 37 °C incubator for 20±2 h;
7.2.3 After incubation, the EP tubes were placed in a centrifugal machine, plasma samples were collected by centrifugation at 5000 rpm for 10 min, and the IL-6 secretion level was determined;

### 7.3 Determination of IL-6 secretion level in plasma

IL-6 ELISA Kit (Xiamen Innovax Biotech CO., LTD.) was used to determine the IL-6 level in plasma.

The method for determining IL-6 was the same as the one described in Example 4.

The results were shown in Figs. 7A-7B. The actually changed IL-6 levels, resulted from the stimulations of whole blood of subjects with RV0183 and Plcd, had complementary effects in the screening of aTB patients. ROC analysis was further used to test the effects of using RV0183 or PlcD alone, or using RV0183 and PlcD in combination in the diagnosis of active tuberculosis. ROC curve showed that the use of the combination of RV0183 and PlcD in assay could enhance the sensitivity and specificity of the assay, and the result was shown in Fig. 8.

As seen from the results above, the actually changed IL-6 levels, resulted from the stimulation with RV0183 or Plcd, could be used alone or in combination for the detection of aTB patients, and had high sensitivity and specificity.

### Example 8. Evaluation of the ability of other antigens of M. tuberculosis to screen or diagnose of active tuberculosis

### 8.1 Cohort subject information

Whole blood samples were collected from 5 patients who were clinically diagnosed with active tuberculosis, and 5 latently infected individuals who had no clinical symptoms.

### 8.2 Sample collection and the system for Sample stimulation culture

8.2.1 10 ml whole blood sample was collected, and dispensed to 9 EP tubes, at 1 ml whole blood per tube. Among them, 1 EP tube was used as negative control tube (N), to the other 8 EP tubes, one of the 7 antigens as listed in Table 3 was added, at 2 µg per tube;
8.2.2 After dispensing, the whole blood was mixed homogenously by turning the tube upside down, and incubated statically in a 37 °C incubator for 20±2 h;
8.2.3 After incubation, the EP tubes were placed in a centrifugal machine, plasma samples were collected by centrifugation at 5000 rpm for 10 min, and the IL-6 secretion level was determined;

### 8.3 Determination of IL-6 secretion level in plasma

IL-6 ELISA Kit (Xiamen Innovax Biotech CO., LTD.) was used to determine the IL-6 level in plasma.

The method for determining IL-6 was the same as the one described in Example 4.

The result was shown in Table 4. After the stimulation with the antigens such as ESAT-6, CFP-10, RV1009, RV1884c, RV2389c, RV2450c, RV3097c, and RV3542, there was no significant change in the IL-6 level between the aTB samples and the LTBI samples, that is, these antigens could not be used to distinguish active tuberculosis from latent tuberculosis infection.

**Table 4**

| | aTB | aTB | aTB | aTB | aTB | LTBI | LTBI | LTBI | LTBI | LTBI |
|---|---|---|---|---|---|---|---|---|---|---|
| ESAT-6 | 0.085 | 0.162 | 0.132 | 0.203 | 0.162 | 0.051 | 0.069 | 0.097 | 0.071 | 0.125 |
| CFP-10 | 0.038 | 0.074 | 0.139 | 0.12 | 0.074 | 0.044 | 0.075 | 0.1 | 0.079 | 0.061 |
| RV1009 | 0.054 | 0.086 | 0.157 | 0.094 | 0.086 | 0.057 | 0.072 | 0.074 | 0.066 | 0.065 |
| RV1884C | 0.172 | 0.062 | 0.135 | 0.121 | 0.062 | 0.037 | 0.074 | 0.106 | 0.096 | 0.054 |
| RV2389C | 0.205 | 0.053 | 0.282 | 0.132 | 0.053 | 0.081 | 0.065 | 0.1 | 0.198 | 0.055 |
| RV2450C | 0.07 | 0.075 | 0.818 | 0.379 | 0.075 | 0.148 | 0.082 | 0.282 | 0.094 | 0.059 |
| RV3542 | 0.067 | 0.171 | 0.251 | 0.279 | 0.171 | 0.054 | 0.023 | 0.091 | 0.136 | 0.129 |
| RV3097C | 0.073 | 0.067 | 0.032 | 0.207 | 0.067 | 0.064 | 0.136 | 0.086 | 0.284 | 0.057 |

### Example 9. Screening of aTB patients from patients with different pulmonary diseases by using a polypeptide library of RV0183

### 9.1 Cohort subject information

9.1.1 Active TB cases: 101 patients, who were clinically diagnosed with active tuberculosis;
9.1.2 Cases with old tuberculosis: 19 patients with old tuberculosis, who had no clinical symptoms;
9.1.3 Cases with a non-TB pulmonary disease in clinic: the cases, who were tested positive in IGRA, were the individuals latently infected with *M. tuberculosis;* the cases, who were tested negative in IGRA, were the individuals not infected with *M. tuberculosis.*

### 9.2 Sample collection and the system for sample stimulation and culture

9.2.1 Sample collection: Sample collection and treatment were the same as those described in Example 5.
9.2.2 Sample stimulation and culture: a polypeptide library comprising 21 antigenic fragments of RV0183 (SEQ ID NOs: 5-25) was used as a stimulating agent (Ta), to stimulate whole blood. Each polypeptide of the polypeptide library was formulated as a solution at a concentration of 1 µg/mL, and after mixing these solutions in an equal amount, 30 µL was used in the experiment. The other operations were the same as those described in Example 5.

### 9.3 Determination of IL-6 level

The same as Example 5.

The result was shown in Fig. 9. After stimulation of whole blood samples with the polypeptide library of RV0183, the IL-6 level in aTB patients were significantly higher than that in patients with old pulmonary tuberculosis and patients with other pulmonary diseases, indicating that the polypeptide library could be used to screen aTB patients.

Although the specific embodiments of the invention have been described in details, those skilled in the art would understand that, according to all the disclosed teachings, various modifications and changes can be made, and that such modifications and changes are within the scope of the present invention. The scope of the present invention is given by the appended claims and any equivalents thereof.

## Claims

1. A kit comprising one or more of RV0183, PlcD, or antigenic fragments thereof, and
a reagent capable of detecting IL-6;
preferably, the RV0183 has an amino acid sequence set forth in SEQ ID NO: 1; and/or, the PlcD has an amino acid sequence set forth in SEQ ID NO: 3;
preferably, the kit comprises RV0183 and/or PlcD;
preferably, the kit comprises one or more antigenic fragments of RV0183; more preferably, the antigenic fragment has an amino acid sequence selected from: SEQ ID NOs: 5-25;
preferably, the kit comprises antigenic fragments having the amino acid sequences set forth in SEQ ID NOs: 13, 14 and 19; optionally, the kit further comprises a combination of the following antigenic fragments:
1) antigenic fragments having the amino acid sequences set forth in SEQ ID NOs: 5, 11 and 22,
2) antigenic fragments having the amino acid sequences set forth in SEQ ID NOs: 7-8 and 11-12,
3) antigenic fragments having the amino acid sequences set forth in SEQ ID NOs: 5-7, 11-12, 22, and 24, or
4) antigenic fragments having the amino acid sequences set forth in SEQ ID NOs: 5, 8-10, 12, 15, and 22-25;
preferably, the kit comprises antigenic fragments having the amino acid sequences set forth in SEQ ID NOs: 5-25;
preferably, the reagent capable of detecting IL-6 is a substance that can specifically bind to IL-6, for example, an antibody, a targeting polypeptide or an aptamer; optionally, the reagent further comprises a detectable label;
preferably, the reagent determines the level of IL-6 in the sample by immunologic assay; more preferably, the immunologic assay is selected from the group consisting of ELISA assay, Elispot assay, Western blot, and surface plasmon resonance;
preferably, the reagent comprises an anti-IL-6 antibody or an antigen binding fragment thereof; more preferably, the reagent determines the level of IL-6 by ELISA;
preferably, the anti-IL-6 antibody is a monoclonal antibody or a polyclonal antibody;
preferably, the anti-IL-6 antibody is an IgG antibody or an IgM antibody;
preferably, the kit further comprises one or more devices or reagents selected from 1) to 5):
1) a blood collection device, e.g. a pyrogen-free vacuum blood collection tube;
2) an anticoagulant, e.g. heparin;
3) a culture solution or a culture medium;
4) a non-specific stimulating agent, e.g. phytohemagglutinin or Concanavalin A;
5) a diluent, e.g. phosphate buffer or physiological saline;
preferably, the kit is useful for diagnosing active tuberculosis, determining the therapeutic effect of a therapy on active tuberculosis or screening a candidate drug capable of treating active tuberculosis.

2. Use of a specific stimulating agent in the manufacture of a kit for diagnosing active tuberculosis; wherein, the specific stimulating agent is one or more selected from the group consisting of RV0183, PlcD, and antigenic fragments thereof;
preferably, the RV0183 has an amino acid sequence set forth in SEQ ID NO: 1; and/or, the PlcD has an amino acid sequence set forth in SEQ ID NO: 3;
preferably, the specific stimulating agent is selected from the group consisting of RV0183, PlcD, and a combination thereof;
preferably, the specific stimulating agent is selected from one or more antigenic fragments of RV0183; more preferably, the antigenic fragment has an amino acid sequence selected from: SEQ ID NOs: 5-25;
preferably, the specific stimulating agent comprises antigenic fragments having the amino acid sequences set forth in SEQ ID NOs: 13, 14 and 19; optionally, the specific stimulating agent further comprises a combination of the following antigenic fragments:
1) antigenic fragments having the amino acid sequences set forth in SEQ ID NOs: 5, 11 and 22,
2) antigenic fragments having the amino acid sequences set forth in SEQ ID NOs: 7-8 and 11-12,
3) antigenic fragments having the amino acid sequences set forth in SEQ ID NOs: 5-7, 11-12, 22 and 24, or
4) antigenic fragments having the amino acid sequences set forth in SEQ ID NOs: 5, 8-10, 12, 15 and 22-25;
preferably, the specific stimulating agent comprises antigenic fragments having the amino acid sequences set forth in SEQ ID NOs: 5-25;
preferably, the kit comprises a reagent capable of detecting IL-6, such as an antibody, a targeting polypeptide or an aptamer that can specifically bind to IL-6; optionally, the reagent further comprises a detectable label;
preferably, the reagent determines the level of IL-6 in the sample by immunologic assay; more preferably, the immunologic assay is selected from the group consisting of ELISA assay, Elispot assay, Western blot, and surface plasmon resonance;
preferably, the reagent comprises an anti-IL-6 antibody or an antigen binding fragment thereof; more preferably, the reagent determines the level of IL-6 by ELISA;
preferably, the anti-IL-6 antibody is a monoclonal antibody or a polyclonal antibody; preferably, the anti-IL-6 antibody is an IgG antibody or an IgM antibody;
preferably, the kit further comprises one or more devices or reagents selected from 1) to 5):
1) a blood collection device, e.g. a pyrogen-free vacuum blood collection tube;
2) an anticoagulant, e.g. heparin;
3) a culture solution or a culture medium;
4) a non-specific stimulating agent, e.g. phytohemagglutinin or Concanavalin A;
5) a diluent, e.g. phosphate buffer or physiological saline;
preferably, the kit diagnoses whether the subject is suffering from active tuberculosis, by a method comprising the following steps:
(1) stimulating at least one sample from the subject with a specific stimulating agent, and using the at least one sample as a test sample, and using a non-stimulated sample from the subject, as a negative control sample, wherein the specific stimulating agent is one or more selected from the group consisting of RV0183, PlcD, and antigenic fragments thereof;
(2) determining the level of IL-6 in each of the samples in the step (1) by using a reagent capable of detecting IL-6, and calculating a difference value in the level of IL-6 between the test sample and the negative control sample; and
(3) comparing the difference value with a reference value, or subjecting the difference value to statistical analysis so as to obtain a statistical analysis value, and comparing the statistical analysis value with a reference value, and determining whether the subject is suffering from active tuberculosis;
wherein, the sample comprises peripheral blood mononuclear cell (PBMC), for example, whole blood (e.g. anticoagulated whole blood), peripheral blood mononuclear cell (PBMC), or peripheral blood buffy coat;
preferably, in the step (3), statistical analysis of the difference value is carried out by using a statistical model selected from the group consisting of: Linear combination, Linear regression model, Logistic regression model, Linear discrimination analysis (LDA) model, The nearest neighbor model and Prediction Analysis of Microarrays (PAM); more preferably, in the step (3), statistical analysis of the difference value is carried out by using Logistic regression model;
preferably, in the step (1), stimulating one or more samples from the subject with at least two specific stimulating agents together or separately, and using the one or more samples as the test sample, wherein the specific stimulating agents are each independently selected from the group consisting of RV0183, PlcD, and antigenic fragments thereof; more preferably, in the step (1), stimulating at least two samples with RV0183 and PlcD separately, and using the at least two samples as the test sample; or, in the step (1), stimulating at least one sample with one or more antigenic fragments of RV0183 together, and using the at least one sample as the test sample;
preferably, the step (1) further comprises: stimulating at least one sample with a non-specific stimulating agent, and using the at least one sample as a positive control sample; more preferably, the non-specific stimulating agent includes phytohemagglutinin or Concanavalin A;
preferably, prior to the step (1), the method further comprises one or more of the following steps: (a) obtaining the sample from the subject by using a blood collection device; (b) treating the blood collection device or the sample from the subject with an anticoagulant; (c) treating the sample from the subject with a culture solution or a culture medium; and, (d) diluting the sample from the subject with a diluent.

3. Use of a specific stimulating agent in the manufacture of a kit for determining the therapeutic effect of a therapy on active tuberculosis; wherein, the specific stimulating agent is one or more selected from the group consisting of RV0183, PlcD, and antigenic fragments thereof;
preferably, the RV0183 has an amino acid sequence set forth in SEQ ID NO: 1; and/or, the PlcD has an amino acid sequence set forth in SEQ ID NO: 3;
preferably, the specific stimulating agent is selected from the group consisting of RV0183, PlcD, and a combination thereof;
preferably, the specific stimulating agent is selected from one or more antigenic fragments of RV0183; more preferably, the antigenic fragment has an amino acid sequence selected from: SEQ ID NOs: 5-25;
preferably, the specific stimulating agent comprises antigenic fragments having the amino acid sequences set forth in SEQ ID NOs: 13, 14 and 19; optionally, the specific stimulating agent further comprises a combination of the following antigenic fragments:
1) antigenic fragments having the amino acid sequences set forth in SEQ ID NOs: 5, 11 and 22,
2) antigenic fragments having the amino acid sequences set forth in SEQ ID NOs: 7-8 and 11-12,
3) antigenic fragments having the amino acid sequences set forth in SEQ ID NOs: 5-7, 11-12, 22 and 24, or
4) antigenic fragments having the amino acid sequences set forth in SEQ ID NOs: 5, 8-10, 12, 15 and 22-25;
preferably, the specific stimulating agent comprises antigenic fragments having the amino acid sequences set forth in SEQ ID NOs: 5-25;
preferably, the kit comprises a reagent capable of detecting IL-6, such as an antibody, a targeting polypeptide or an aptamer that can specifically bind to IL-6; optionally, the reagent further comprises a detectable label;
preferably, the reagent determines the level of IL-6 in the sample by immunologic assay; more preferably, the immunologic assay is selected from the group consisting of ELISA assay, Elispot assay, Western blot, and surface plasmon resonance;
preferably, the reagent comprises an anti-IL-6 antibody or an antigen binding fragment thereof; more preferably, the reagent determines the level of IL-6 by ELISA;
preferably, the anti-IL-6 antibody is a monoclonal antibody or a polyclonal antibody; preferably, the anti-IL-6 antibody is an IgG antibody or an IgM antibody;
preferably, the kit further comprises one or more devices or reagents selected from 1) to 5):
1) a blood collection device, e.g. a pyrogen-free vacuum blood collection tube;
2) an anticoagulant, e.g. heparin;
3) a culture solution or a culture medium;
4) a non-specific stimulating agent, e.g. phytohemagglutinin or Concanavalin A;
5) a diluent, e.g. phosphate buffer or physiological saline;
preferably, the kit determines the therapeutic effect of a therapy on active tuberculosis by a method comprising the following steps:
(1) before administering the therapy to a subject, obtaining at least two samples from the subject, as a pre-therapy sample;
(2) stimulating at least one pre-therapy sample from the subject with a specific stimulating agent, and using the at least one pre-therapy sample as a test sample, and using at least one non-stimulated pre-therapy sample from the subject, as a negative control sample, wherein the specific stimulating agent is one or more selected from the group consisting of RV0183, PlcD, and antigenic fragments thereof;
(3) determining the level of IL-6 in each of the samples in the step (2) by using a reagent capable of detecting IL-6, and calculating a difference value in the level of IL-6 between the test sample and the negative control sample, as a first difference value;
(4) administering the therapy to the subject;
(5) after administering the therapy to the subject, obtaining at least two samples from the subject, as a post-therapy sample;
(6) stimulating at least one post-therapy sample from the subject with a specific stimulating agent, and using the at least one post-therapy sample as a test sample, and using at least one non-stimulated post-therapy sample from the subject, as a negative control sample, wherein the specific stimulating agent is one or more selected from the group consisting of RV0183, PlcD, and antigenic fragments thereof;
(7) determining the level of IL-6 in each of the samples in the step (6) by using a reagent capable of detecting IL-6, and calculating a difference value in the level of IL-6 between the test sample and the negative control sample, as a second difference value; and
(8) comparing the second difference value with the first difference value, or separately subjecting the first difference value and the second difference value to statistical analysis so as to obtain a statistical analysis value of the first difference value and a statistical analysis value of the second difference value, and comparing the statistical analysis value of the second difference value with the statistical analysis value of the first difference value, and determining whether the therapy is effective in the treatment of active tuberculosis;
wherein, the sample comprises peripheral blood mononuclear cell (PBMC), for example, whole blood (e.g. anticoagulated whole blood), peripheral blood mononuclear cell (PBMC), or peripheral blood buffy coat;
preferably, in the step (8), statistical analysis of the first difference value and the second difference value is carried out by using a statistical model selected from the group consisting of: Linear combination, Linear regression model, Logistic regression model, Linear discrimination analysis (LDA) model, The nearest neighbor model and Prediction Analysis of Microarrays (PAM); more preferably, in the step (8), statistical analysis of the first difference value and the second difference value is carried out by using Logistic regression model;
preferably, in the steps (2) and (6), the pre-therapy sample and the post-therapy sample are subjected to the same treatment; preferably, in the steps (2) and (6), stimulating one or more samples from the subject with at least two specific stimulating agents together or separately, and using the one or more samples as the test sample, wherein the specific stimulating agents are each independently selected from the group consisting of RV0183, PlcD, and antigenic fragments thereof; more preferably, in the steps (2) and (6), stimulating at least two samples with RV0183 and PlcD separately, and using the at least two samples as the test sample; or, in the steps (2) and (6), stimulating at least one sample with one or more antigenic fragments of RV0183 together, and using the at least one sample as the test sample;
preferably, the subject is a mammal, such as human;
preferably, the therapy comprises administering an antitubercular agent to the subject, such as isoniazid, rifampicin, streptomycin, pyrazinamide, ethambutol or any combination thereof;
preferably, the steps (2) and (6) further comprise: stimulating at least one sample with a non-specific stimulating agent, and using the at least one sample as a positive control sample; more preferably, the non-specific stimulating agent includes phytohemagglutinin or Concanavalin A;
preferably, in the step (1), the pre-therapy sample from the subject is obtained by using a blood collection device;
preferably, in the step (5), the post-therapy sample from the subject is obtained by using a blood collection device;
preferably, prior to the step (1), the method further comprises one or more of the following steps: (a) treating the blood collection device or the sample from the subject with an anticoagulant; (b) treating the sample from the subject with a culture solution or a culture medium; and, (c) diluting the sample from the subject with a diluent;
preferably, prior to the step (5), the method further comprises one or more of the following steps: (a) treating the blood collection device or the sample from the subject with an anticoagulant; (b) treating the sample from the subject with a culture solution or a culture medium; and, (c) diluting the sample from the subject with a diluent.

4. Use of a specific stimulating agent in the manufacture of a kit for screening a candidate drug capable of treating active tuberculosis; wherein, the specific stimulating agent is one or more selected from the group consisting of RV0183, PlcD, and antigenic fragments thereof;
preferably, the RV0183 has an amino acid sequence set forth in SEQ ID NO: 1; and/or, the PlcD has an amino acid sequence set forth in SEQ ID NO: 3;
preferably, the specific stimulating agent is selected from the group consisting of RV0183, PlcD, and a combination thereof;
preferably, the specific stimulating agent is selected from one or more antigenic fragments of RV0183; more preferably, the antigenic fragment has an amino acid sequence selected from: SEQ ID NOs: 5-25;
preferably, the specific stimulating agent comprises antigenic fragments having the amino acid sequences set forth in SEQ ID NOs: 13, 14 and 19; optionally, the specific stimulating agent further comprises a combination of the following antigenic fragments:
1) antigenic fragments having the amino acid sequences set forth in SEQ ID NOs: 5, 11 and 22,
2) antigenic fragments having the amino acid sequences set forth in SEQ ID NOs: 7-8 and 11-12,
3) antigenic fragments having the amino acid sequences set forth in SEQ ID NOs: 5-7, 11-12, 22 and 24, or
4) antigenic fragments having the amino acid sequences set forth in SEQ ID NOs: 5, 8-10, 12, 15 and 22-25;
preferably, the specific stimulating agent comprises antigenic fragments having the amino acid sequences set forth in SEQ ID NOs: 5-25;
preferably, the kit comprises a reagent capable of detecting IL-6, such as an antibody, a targeting polypeptide or an aptamer that can specifically bind to IL-6; optionally, the reagent further comprises a detectable label;
preferably, the reagent determines the level of IL-6 in the sample by immunologic assay; more preferably, the immunologic assay is selected from the group consisting of ELISA assay, Elispot assay, Western blot, and surface plasmon resonance;
preferably, the reagent comprises an anti-IL-6 antibody or an antigen binding fragment thereof; more preferably, the reagent determines the level of IL-6 by ELISA;
preferably, the anti-IL-6 antibody is a monoclonal antibody or a polyclonal antibody; preferably, the anti-IL-6 antibody is an IgG antibody or an IgM antibody;
preferably, the kit further comprises one or more devices or reagents selected from 1) to 5):
1) a blood collection device, e.g. a pyrogen-free vacuum blood collection tube;
2) an anticoagulant, e.g. heparin;
3) a culture solution or a culture medium;
4) a non-specific stimulating agent, e.g. phytohemagglutinin or Concanavalin A;
5) a diluent, e.g. phosphate buffer or physiological saline;
preferably, the kit screens a candidate drug capable of treating active tuberculosis by a method comprising the following steps:
(1) before administering the candidate drug to an model animal, obtaining at least two samples from the animal, as a pre-therapy sample;
(2) stimulating at least one pre-therapy sample from the animal with a specific stimulating agent, and using the at least one pre-therapy sample as a test sample, and using at least one non-stimulated pre-therapy sample from the animal, as a negative control sample, wherein the specific stimulating agent is one or more selected from the group consisting of RV0183, PlcD, and antigenic fragments thereof;
(3) determining the level of IL-6 in each of the samples in the step (2) by using a reagent capable of detecting IL-6, and calculating a difference value in the level of IL-6 between the test sample and the negative control sample, as a first difference value;
(4) administering the candidate drug to the animal;
(5) after administering the candidate drug to the animal, obtaining at least two samples from the animal, as a post-therapy sample;
(6) stimulating at least one post-therapy sample from the animal with a specific stimulating agent, and using the at least one post-therapy sample as a test sample, and using at least one non-stimulated post-therapy sample from the animal, as a negative control sample, wherein the specific stimulating agent is one or more selected from the group consisting of RV0183, PlcD, and antigenic fragments thereof;
(7) determining the level of IL-6 in each of the samples in the step (6) by using a reagent capable of detecting IL-6, and calculating a difference value in the level of IL-6 between the test sample and the negative control sample, as a second difference value; and
(8) comparing the second difference value with the first difference value, or separately subjecting the first difference value and the second difference value to statistical analysis so as to obtain a statistical analysis value of the first difference value and a statistical analysis value of the second difference value, and comparing the statistical analysis value of the second difference value with the statistical analysis value of the first difference value, and determining whether the therapy is effective in the treatment of active tuberculosis;
wherein, the sample comprises peripheral blood mononuclear cell (PBMC), for example, whole blood (e.g. anticoagulated whole blood), peripheral blood mononuclear cell (PBMC), or peripheral blood buffy coat;
preferably, in the step (8), statistical analysis of the first difference value and the second difference value is carried out by using a statistical model selected from the group consisting of: Linear combination, Linear regression model, Logistic regression model, Linear discrimination analysis (LDA) model, The nearest neighbor model and Prediction Analysis of Microarrays (PAM); more preferably, in the step (8), statistical analysis of the first difference value and the second difference value is carried out by using Logistic regression model.
preferably, in the steps (2) and (6), the pre-therapy sample and the post-therapy sample are subjected to the same treatment; preferably, in the steps (2) and (6), stimulating one or more samples from the animal with at least two specific stimulating agents together or separately, and using the one or more samples as the test sample, wherein the specific stimulating agents are each independently selected from the group consisting of RV0183, PlcD, and antigenic fragments thereof; more preferably, in the steps (2) and (6), stimulating at least two samples with RV0183 and PlcD separately, and using the at least two samples as the test sample; or, in the steps (2) and (6), stimulating at least one sample with one or more antigenic fragments of RV0183 together, and using the at least one sample as the test sample;
preferably, the model animal is a non-human mammal, for example, a mouse, a guinea pig, a rabbit or a non-human primate (e.g. a cynomolgus monkey or a rhesus monkey);
preferably, the subject is a mammal, such as human;
preferably, the steps (2) and (6) further comprise: stimulating at least one sample with a non-specific stimulating agent, and using the at least one sample as a positive control sample; more preferably, the non-specific stimulating agent includes phytohemagglutinin or Concanavalin A;
preferably, in the step (1), the pre-therapy sample from the animal is obtained by using a blood collection device;
preferably, in the step (5), the post-therapy sample from the animal is obtained by using a blood collection device;
preferably, prior to the step (1), the method further comprises one or more of the following steps: (a) treating the blood collection device or the sample from the animal with an anticoagulant; (b) treating the sample from the animal with a culture solution or a culture medium; and, (c) diluting the sample from the animal with a diluent;
preferably, prior to the step (5), the method further comprises one or more of the following steps: (a) treating the blood collection device or the sample from the animal with an anticoagulant; (b) treating the sample from the animal with a culture solution or a culture medium; and, (c) diluting the sample from the animal with a diluent.

5. A method for diagnosing whether a subject is suffering from active tuberculosis, comprising the following steps:
(1) providing at least two samples from the subject;
(2) stimulating at least one sample from the subject with a specific stimulating agent, and using the at least one sample as a test sample, and using a non-stimulated sample, as a negative control sample, wherein the specific stimulating agent is one or more selected from the group consisting of RV0183, PlcD, and antigenic fragments thereof;
(3) determining the level of IL-6 in each of the samples in the step (2), and calculating a difference value in the level of IL-6 between the test sample and the negative control sample; and
(4) comparing the difference value with a reference value, or subjecting the difference value to statistical analysis so as to obtain a statistical analysis value, and comparing the statistical analysis value with a reference value, and determining whether the subject is suffering from active tuberculosis;
wherein, the sample comprises peripheral blood mononuclear cell (PBMC), for example, whole blood (e.g. anticoagulated whole blood), peripheral blood mononuclear cell (PBMC), or peripheral blood buffy coat;
preferably, in the step (4), the difference value is subjected to statistical analysis by using a statistical model selected from the group consisting of: Linear combination, Linear regression model, Logistic regression model, Linear discrimination analysis (LDA) model, The nearest neighbor model and Prediction Analysis of Microarrays (PAM); more preferably, in the step (4), statistical analysis of the difference value is carried out by using Logistic regression model;
preferably, the RV0183 has an amino acid sequence set forth in SEQ ID NO: 1; and/or, the PlcD has an amino acid sequence set forth in SEQ ID NO: 3;
preferably, the antigenic fragments are antigenic fragments of RV0183; more preferably, the antigenic fragment has an amino acid sequence selected from: SEQ ID NOs: 5-25;
preferably, the subject is a mammal, such as human;
preferably, in the step (2), stimulating one or more samples from the subject with at least two specific stimulating agents together or separately, and using the one or more samples as the test sample, wherein the specific stimulating agents are each independently selected from the group consisting of RV0183, PlcD, and antigenic fragments thereof;
preferably, in the step (2), stimulating at least two samples with RV0183 and PlcD separately, and using the at least two samples as the test sample; or, in the step (2), stimulating at least one sample with one or more of the antigenic fragments together, and using the at least one sample as the test sample; more preferably, in the step (2), stimulating at least one sample with a combination of the following antigenic fragments together, and using the at least one sample as the test sample:
1) antigenic fragments having the amino acid sequences set forth in SEQ ID NOs: 13, 14 and 19,
2) antigenic fragments having the amino acid sequences set forth in SEQ ID NOs: 5, 11, 13-14, 19 and 22,
3) antigenic fragments having the amino acid sequences set forth in SEQ ID NOs: 7-8, 11-14 and 19,
4) antigenic fragments having the amino acid sequences set forth in SEQ ID NOs: 5-7, 11-14, 19, 22 and 24,
5) antigenic fragments having the amino acid sequences set forth in SEQ ID NOs: 5, 8-10, 12-15, 19 and 22-25; or
6) antigenic fragments having the amino acid sequences set forth in SEQ ID NOs: 5-25;
preferably, in the step (3), the level of IL-6 in the samples is determined by immunologic assay; more preferably, the immunologic assay is selected from the group consisting of ELISA assay, Elispot assay, Western blot, and surface plasmon resonance;
preferably, in the step (3), the level of IL-6 is determined by using an anti-IL-6 antibody or an antigen binding fragment thereof, for example, by ELISA;
preferably, the anti-IL-6 antibody is a monoclonal antibody or a polyclonal antibody; preferably, the anti-IL-6 antibody is an IgG antibody or an IgM antibody;
preferably, the step (2) further comprises: stimulating at least one sample with a non-specific stimulating agent, and using the at least one sample as a positive control sample; more preferably, the non-specific stimulating agent includes phytohemagglutinin or Concanavalin A;
preferably, prior to the step (1), the method further comprises one or more of the following steps: (a) obtaining the sample from the subject; (b) adding an anticoagulant such as heparin to the sample; (c) obtaining PBMC or a PBMC-containing blood component (e.g. peripheral blood buffy coat) from the sample; (d) adding a culture solution or a culture medium into the sample; and (e) diluting the sample.

6. A method for determining the therapeutic effect of a therapy on active tuberculosis, comprising the following steps:
(1) before administering the therapy to a subject, obtaining at least two samples from the subject, as a pre-therapy sample;
(2) stimulating at least one pre-therapy sample from the subject with a specific stimulating agent, and using the at least one pre-therapy sample as a test sample, and using at least one non-stimulated pre-therapy sample from the subject, as a negative control sample, wherein the specific stimulating agent is one or more selected from the group consisting of RV0183, PlcD, and antigenic fragments thereof;
(3) determining the level of IL-6 in each of the samples in the step (2), and calculating a difference value in the level of IL-6 between the test sample and the negative control sample, as a first difference value;
(4) administering the therapy to the subject;
(5) after administering the therapy to the subject, obtaining at least two samples from the subject, as a post-therapy sample;
(6) stimulating at least one post-therapy sample from the subject with a specific stimulating agent, and using the at least one post-therapy sample as a test sample, and using at least one non-stimulated post-therapy sample from the subject, as a negative control sample, wherein the specific stimulating agent is one or more selected from the group consisting of RV0183, PlcD, and antigenic fragments thereof;
(7) determining the level of IL-6 in each of the samples in the step (6) by using a reagent capable of detecting IL-6, and calculating a difference value in the level of IL-6 between the test sample and the negative control sample, as a second difference value; and
(8) comparing the second difference value with the first difference value, or separately subjecting the first difference value and the second difference value to statistical analysis so as to obtain a statistical analysis value of the first difference value and a statistical analysis value of the second difference value, and comparing the statistical analysis value of the second difference value with the statistical analysis value of the first difference value, and determining whether the therapy is effective in the treatment of active tuberculosis;
wherein, the sample comprises peripheral blood mononuclear cell (PBMC), for example, whole blood (e.g. anticoagulated whole blood), peripheral blood mononuclear cell (PBMC), or peripheral blood buffy coat;
preferably, in the step (8), statistical analysis of the first difference value and the second difference value is carried out by using a statistical model selected from the group consisting of: Linear combination, Linear regression model, Logistic regression model, Linear discrimination analysis (LDA) model, The nearest neighbor model and Prediction Analysis of Microarrays (PAM); more preferably, in the step (8), statistical analysis of the first difference value and the second difference value is carried out by using Logistic regression model;
preferably, the RV0183 has an amino acid sequence set forth in SEQ ID NO: 1; and/or, the PlcD has an amino acid sequence set forth in SEQ ID NO: 3;
preferably, the antigenic fragments are antigenic fragments of RV0183; more preferably, the antigenic fragment has an amino acid sequence selected from: SEQ ID NOs: 5-25;
preferably, the subject is a mammal, such as human;
preferably, the therapy comprises administering an antitubercular agent to the subject, for example, isoniazid, rifampicin, streptomycin, pyrazinamide, ethambutol or any combination thereof;
preferably, in the steps (2) and (6), the pre-therapy sample and the post-therapy sample are subjected to the same treatment; preferably, in the steps (2) and (6), stimulating one or more samples from the subject with at least two specific stimulating agents together or separately, and using the one or more samples as the test sample, wherein the specific stimulating agents are each independently selected from the group consisting of RV0183, PlcD, and antigenic fragments thereof;
preferably, in the steps (2) and (6), stimulating at least two samples with RV0183 and PlcD separately, and using the at least two samples as the test sample; or, in the steps (2) and (6), stimulating at least one sample with one or more of the antigenic fragments together, and using the at least one sample as the test sample; more preferably, in the steps (2) and (6), stimulating at least one sample with a combination of the following antigenic fragments together, and using the at least one sample as the test sample:
1) antigenic fragments having the amino acid sequences set forth in SEQ ID NOs: 13, 14 and 19,
2) antigenic fragments having the amino acid sequences set forth in SEQ ID NOs: 5, 11, 13-14, 19 and 22,
3) antigenic fragments having the amino acid sequences set forth in SEQ ID NOs: 7-8, 11-14 and 19,
4) antigenic fragments having the amino acid sequences set forth in SEQ ID NOs: 5-7, 11-14, 19, 22 and 24,
5) antigenic fragments having the amino acid sequences set forth in SEQ ID NOs: 5, 8-10, 12-15, 19 and 22-25; or
6) antigenic fragments having the amino acid sequences set forth in SEQ ID NOs: 5-25;
preferably, in the step (3), the level of IL-6 in the samples is determined by immunologic assay; more preferably, the immunologic assay is selected from the group consisting of ELISA assay, Elispot assay, Western blot, and surface plasmon resonance;
preferably, in the step (3), the level of IL-6 is determined by using an anti-IL-6 antibody or an antigen binding fragment thereof, for example, by ELISA;
preferably, the anti-IL-6 antibody is a monoclonal antibody or a polyclonal antibody; preferably, the anti-IL-6 antibody is an IgG antibody or an IgM antibody;
preferably, the steps (2) and (6) further comprise: stimulating at least one sample with a non-specific stimulating agent, and using the at least one sample as a positive control sample; more preferably, the non-specific stimulating agent includes phytohemagglutinin or Concanavalin A;
preferably, prior to the step (1), the method further comprises one or more of the following steps: (a) adding an anticoagulant such as heparin into the pre-therapy sample; (b) obtaining PBMC or a PBMC-containing blood component (e.g. peripheral blood buffy coat) from the pre-therapy sample; (c) adding a culture solution or a culture medium into the pre-therapy sample; and, (d) diluting the pre-therapy sample;
preferably, prior to the step (5), the method further comprises one or more of the following steps: (a) adding an anticoagulant such as heparin to the post-therapy sample; (b) obtaining PBMC or a PBMC-containing blood component (e.g. peripheral blood buffy coat) from the post-therapy sample; (c) adding a culture solution or a culture medium to the post-therapy sample; and, (d) diluting the post-therapy sample.

7. A method for screening a candidate drug capable of treating active tuberculosis, comprising the following steps:
(1) before administering the candidate drug to an model animal, obtaining at least two samples from the animal, as a pre-therapy sample;
(2) stimulating at least one pre-therapy sample from the animal with a specific stimulating agent, and using the at least one pre-therapy sample as a test sample, and using at least one non-stimulated pre-therapy sample from the animal, as a negative control sample, wherein the specific stimulating agent is one or more selected from the group consisting of RV0183, PlcD, and antigenic fragments thereof;
(3) determining the level of IL-6 in each of the samples in the step (2), and calculating a difference value in the level of IL-6 between the test sample and the negative control sample, as a first difference value;
(4) administering the candidate drug to the animal;
(5) after administering the candidate drug to the animal, obtaining at least two samples from the animal, as a post-therapy sample;
(6) stimulating at least one post-therapy sample from the animal with a specific stimulating agent, and using the at least one post-therapy sample as a test sample, and using at least one non-stimulated post-therapy sample from the animal, as a negative control sample, wherein the specific stimulating agent is one or more selected from the group consisting of RV0183, PlcD, and antigenic fragments thereof;
(7) determining the level of IL-6 in each of the samples in the step (6) by using a reagent capable of detecting IL-6, and calculating a difference value in the level of IL-6 between the test sample and the negative control sample, as a second difference value; and
(8) comparing the second difference value with the first difference value, or separately subjecting the first difference value and the second difference value to statistical analysis so as to obtain a statistical analysis value of the first difference value and a statistical analysis value of the second difference value, and comparing the statistical analysis value of the second difference value with the statistical analysis value of the first difference value, and determining whether the therapy is effective in the treatment of active tuberculosis;
wherein, the sample comprises peripheral blood mononuclear cell (PBMC), for example, whole blood (e.g. anticoagulated whole blood), peripheral blood mononuclear cell (PBMC), or peripheral blood buffy coat;
preferably, in the step (8), statistical analysis of the first difference value and the second difference value is carried out by using a statistical model selected from the group consisting of: Linear combination, Linear regression model, Logistic regression model, Linear discrimination analysis (LDA) model, The nearest neighbor model and Prediction Analysis of Microarrays (PAM); more preferably, in the step (8), statistical analysis of the first difference value and the second difference value is carried out by using Logistic regression model;
preferably, the RV0183 has an amino acid sequence set forth in SEQ ID NO: 1; and/or, the PlcD has an amino acid sequence set forth in SEQ ID NO: 3;
preferably, the antigenic fragments are antigenic fragments of RV0183; more preferably, the antigenic fragment has an amino acid sequence selected from: SEQ ID NOs: 5-25;
preferably, the model animal is a non-human mammal, for example, a mouse, a guinea pig, a rabbit or a non-human primate (e.g. a cynomolgus monkey or a rhesus monkey);
preferably, the subject is a mammal, such as human;
preferably, in the steps (2) and (6), the pre-therapy sample and the post-therapy sample are subjected to the same treatment; preferably, in the steps (2) and (6), stimulating one or more samples from the animal with at least two specific stimulating agents together or separately, and using the one or more samples as the test sample, wherein the specific stimulating agents are each independently selected from the group consisting of RV0183, PlcD, and antigenic fragments thereof;
preferably, in the steps (2) and (6), stimulating at least two samples with RV0183 and PlcD separately, and using the at least two samples as the test sample; or, in the steps (2) and (6), stimulating at least one sample with one or more of the antigenic fragments together, and using the at least one sample as the test sample; more preferably, in the steps (2) and (6), stimulating at least one sample with a combination of the following antigenic fragments together, and using the at least one sample as the test sample:
1) antigenic fragments having the amino acid sequences set forth in SEQ ID NOs: 13, 14 and 19,
2) antigenic fragments having the amino acid sequences set forth in SEQ ID NOs: 5, 11, 13-14, 19 and 22,
3) antigenic fragments having the amino acid sequences set forth in SEQ ID NOs: 7-8, 11-14 and 19,
4) antigenic fragments having the amino acid sequences set forth in SEQ ID NOs: 5-7, 11-14, 19, 22 and 24,
5) antigenic fragments having the amino acid sequences set forth in SEQ ID NOs: 5, 8-10, 12-15, 19 and 22-25; or
6) antigenic fragments having the amino acid sequences set forth in SEQ ID NOs: 5-25;
preferably, in the step (3), the level of IL-6 in the samples is determined by immunologic assay; more preferably, the immunologic assay is selected from the group consisting of ELISA assay, Elispot assay, Western blot, and surface plasmon resonance;
preferably, in the step (3), the level of IL-6 is determined by using an anti-IL-6 antibody or an antigen binding fragment thereof, for example, by ELISA;
preferably, the anti-IL-6 antibody is a monoclonal antibody or a polyclonal antibody; preferably, the anti-IL-6 antibody is an IgG antibody or an IgM antibody;
preferably, the steps (2) and (6) further comprise: stimulating at least one sample with a non-specific stimulating agent, and using the at least one sample as a positive control sample; more preferably, the non-specific stimulating agent includes phytohemagglutinin or Concanavalin A;
preferably, prior to the step (1), the method further comprises one or more of the following steps: (a) adding an anticoagulant such as heparin into the pre-therapy sample; (b) obtaining PBMC or a PBMC-containing blood component (e.g. peripheral blood buffy coat) from the pre-therapy sample; (c) adding a culture solution or a culture medium into the pre-therapy sample; and, (d) diluting the pre-therapy sample; and;
preferably, prior to the step (5), the method further comprises one or more of the following steps: (a) adding an anticoagulant such as heparin into the post-therapy sample; (b) obtaining PBMC or a PBMC-containing blood component (e.g. peripheral blood buffy coat) from the post-therapy sample; (c) adding a culture solution or a culture medium into the post-therapy sample; and, (d) diluting the post-therapy sample.

8. A polypeptide library, comprising:
a first peptide having an amino acid sequence set forth in SEQ ID NO: 13;
a second peptide having an amino acid sequence set forth in SEQ ID NO: 14; and
a third peptide having an amino acid sequence set forth in SEQ ID NO: 19;
optionally, the polypeptide library further comprises a combination of the following polypeptides:
1) polypeptides having the amino acid sequences set forth in SEQ ID NOs: 5, 11 and 22,
2) polypeptides having the amino acid sequences set forth in SEQ ID NOs: 7-8 and 11-12,
3) polypeptides having the amino acid sequences set forth in SEQ ID NOs: 5-7, 11-12, 22 and 24, or
4) polypeptides having the amino acid sequences set forth in SEQ ID NOs: 5, 8-10, 12, 15 and 22-25;
preferably, the polypeptide library comprises antigenic fragments having the amino acid sequences set forth in SEQ ID NOs: 5-25;
preferably, the polypeptide library can induce the generation of IL-6 in a sample; wherein, the sample comprises peripheral blood mononuclear cell (PBMC), for example, whole blood (e.g. anticoagulated whole blood), peripheral blood mononuclear cell (PBMC), or peripheral blood buffy coat;
preferably, the polypeptide library is useful for diagnosing active tuberculosis, determining the therapeutic effect of a therapy on active tuberculosis or screening a candidate drug capable of treating active tuberculosis.
